# EUROPÄISCHE PATENTSCHRIFT

(11) **EP 1 827 379 B1**
(45) Veröffentlichungstag und Bekanntmachung des Hinweises auf die Patenterteilung: **15.12.2010**
(21) Anmeldenummer: 05795350.7
(22) Anmeldetag: 19.10.2005
(51) Int. Cl.: A61K 9/127, A61K 31/685, C07F 9/10

(54) **FORMULIERUNGEN MIT ALKYLPHOSPHOCHOLINEN UNTER VERWENDUNG VON NEUEN NEGATIVEN LADUNGSTRÄGERN**
FORMULATIONS CONTAINING ALKYLPHOSPHOCHOLINES USING NOVEL NEGATIVE CHARGE CARRIERS
FORMULATIONS CONTENANT DES ALKYLPHOSPHOCHOLINES FAISANT INTERVENIR DE NOUVEAUX PORTEURS DE CHARGE NEGATIFS

(30) Priorität: 19.10.2004 DE 102004050910; 16.11.2004 DE 102004055284
(43) Veröffentlichungstag der Anmeldung: 05.09.2007
(73) Patentinhaber: MPG Max-Planck-Gesellschaft zur Förderung der Wissenschaften e.V., 80539 München (DE)
(72) Erfinder: EIBL, Hansjörg, 37120 Bovenden (DE); WIELAND-BERGHAUSEN, Susanne, Christine, 79541 Lörrach (DE); STEFFAN, Jean, F-68200 Mulhouse (FR)
(74) Vertreter: Dey, Michael
(86) Internationale Anmeldenummer: PCT/EP2005/011252
(87) Internationale Veröffentlichungsnummer: WO 2006/042751

(56) Entgegenhaltungen:
- EP-A1- 0 615 746
- WO-A-96/11670
- WO-A2-01/72289
- WO-A2-03/028736
- DE-A1- 4 132 345
- DUZGUNES N ET AL: "PROTON-INDUCED FUSION OF OLEIC-ACID-PHOSPHATIDYLETHANOLAMINE LIPOSOMES" BIOCHEMISTRY, Bd. 24, Nr. 13, 1985, Seiten 3091-3098, XP002419984 ISSN: 0006-2960
- CONNOR J ET AL: "PH SENSITIVE LIPOSOMES ACID INDUCED LIPOSOME FUSION" PROCEEDINGS OF THE NATIONAL ACADEMY OF SCIENCES OF THE UNITED STATES OF AMERICA, Bd. 81, Nr. 6, 1984, Seiten 1715-1718, XP002419985 ISSN: 0027-8424

## Beschreibung

Die vorliegende Erfindung betrifft neue Arzneimittelformulierungen, welche Alkylphosphocholinverbindungen als Wirkstoffe und gleichzeitig als integrale Bestandteile von Liposomen enthalten. Die Arzneimittelformulierungen sind besonders zur Behandlung oder/und Prophylaxe von Tumorerkrankungen, von Erkrankungen, die durch rasch proliferierende Zellen verursacht werden, und von Protozoenerkrankungen, insbesondere von Leishmaniosen und Amöbenerkrankungen, von Akarinosis und Erkrankungen, die durch Arthropoden verursacht werden, geeignet. Diese Formulierungen können aber auch erfolgreich zur Behandlung von bakteriellen Erkrankungen, wie z.B. Ehrlichiose, herangezogen werden. Auch Augenerkrankungen, die mit unkontrollierten zellulären Prozessen einhergehen, können mit diesen Formulierungen erfolgreich behandelt werden.

Alkylphosphocholinverbindungen haben eine antiproliferative Wirkung und weisen eine gute Wirksamkeit gegen Tumor- und Protozoenerkrankungen auf. Ein großer Nachteil dieser Verbindungen ist jedoch zum einen, dass besonders die Verbindungen mit längerkettigen Kohlenwasserstoffresten eine schlechte Löslichkeit in wässrigen Lösungen aufweisen, was sie für eine intravenöse (I.V.) Verabreichung und auch für eine orale Verabreichung in Form von Trinklösungen ungeeignet macht. Weiterhin werden die Verbindungen bei oraler Verabreichung oftmals nur schlecht oder überhaupt nicht resorbiert. Zum anderen gehen viele dieser wirksamen Verbindungen mit beträchtlichen Nebenwirkungen einher, sodass sie nicht über längere Zeit in hohen Dosen verabreicht werden können. Die Nebenwirkungen der Toxizität beruhen in großem Maße auf der hämolytischen Wirkung der Verbindungen.

Protozoen sind einzellige Lebewesen, von denen einige pathogene Parasiten sind. Zu den häufigsten Vertretern, die den Menschen befallen, gehören Plasmodien (Malaria), Trypanosomen (Schlafkrankheit), Amöben, z.B. Entamöben und Acanthamöben (Amöbenruhr, Encephalitis), und Leishmanien (Leishmaniose).

Als Leishmaniosen werden verschiedene Tropenkrankheiten bezeichnet, welche durch Protozoen der Gattung Leishmania hervorgerufen und durch Blut saugende Insekten übertragen werden. Zurzeit sind drei Leishmania-Arten bekannt, welche sehr unterschiedliche Krankheitsbilder hervorrufen: "Kala-Azar" mit Affektion von Milz und Leber, die "Orientbeule" mit entzündlichen Reaktionen an der Haut und "Espundia" mit Erscheinungen auch an den Schleimhäuten des oberen Atem- und Verdauungstraktes. Der Verlauf aller drei Krankheiten ist weniger charakteristisch als bei anderen Protozoenerkrankungen und verläuft vielfach schleichend. Die Inkubationszeit kann Wochen und sogar Monate betragen. In unbehandelten Fällen werden oft sehr hohe Mortalitätsraten beobachtet.

Die Therapie von Leishmaniosen stützt sich im Wesentlichen noch auf altbekannte Antimonpräparate, vor allem Stibogluconat-Natrium (Pentostam). Die Behandlung wird meist über zwei bis drei Wochen durchgeführt, muss dann jedoch für eine bis zwei Wochen unterbrochen werden, weil häufige Nebenwirkungen sonst ein bedrohliches Ausmaß erreichen und irreversibel werden könnten. Die Nebenwirkungen umfassen Magen-Darm-Reizungen, Kreislaufstörungen bis zum Schock und Leberparenchymschädigung. Als weiterer Nachteil hat sich herausgestellt, dass es bereits Leishmania-Stämme gibt, welche Antimon-resistent sind. Als weitere Pharmaka werden aromatische Diamidine, Pentamidin und Amphotericin B eingesetzt. Diese Mittel werden allerdings meist nur in Kombination mit Antimonverbindungen eingesetzt, darüber hinaus weisen sie ebenfalls beträchtliche Nebenwirkungen auf.

Als Amöben, die den Menschen befallen können, sind insbesondere die folgend genannten von Bedeutung. *Entamoeba histolytica* verursacht beim Menschen Dysenterien und Leberabszesse. Der Erreger ist in vielen Ländern dieser Welt sehr häufig, er verursacht etwa 36 bis 50 Millionen Erkrankungsfälle pro Jahr mit zwischen 40.000 und 110.000 Todesfällen. Der Lebenszyklus ist einfach, die Infektion erfolgt über Zysten, die mit verunreinigtem Wasser oder verunreinigten Nahrungsmitteln aufgenommen werden. Die Zysten passieren den Magen unverändert und existieren im Dickdarm, wobei aus jeder Zyste vier Trophozoiten, die eigentlichen Amöben, entstehen. Im Enddarm enzystiert sich wieder ein Teil der Trophozoiten und bildet so die Dauerformen aus, die außerhalb des Menschen überleben können. Im Dickdarm können die Trophozoiten einerseits leben, ohne großen Schaden anzurichten, aber sie können auch die Darmwand angreifen. Dabei können kleine Schleimhautläsionen, aber auch massiv blutende Ulcera entstehen. Die Folge sind blutige Durchfälle, das Vollbild der Amöbendysenterie. Eine weitere häufige Manifestation der Amöbiasis ist der Amöbenleberabzsess. Hier dringen die Amöben aus dem Darm durch die Mesenterialgefäße in die Leber vor und erzeugen dort große Abszesse. Sowohl der Amöbenleberabszess als auch die intestinale Amöbiasis sind unbehandelt massiv lebensbedrohend.

*E. histolytica* Throphozoiten können ohne den menschlichen Wirt nicht überleben. Im Gegensatz dazu gibt es frei lebende Amöben, die in seltenen Fällen im Menschen ernstere Erkrankungen hervorrufen können. Acanthamoeben (z.B. *Acanthamoeba castellanii, Acanthamoeba culbertsoni)* können bei Immunsupprimierten eine chronische granulomatöse Enzephalitis verursachen, außerdem gibt es relativ häufig Acanthamoeba-Keratitisfälle bei Kontaktlinsenträgern. *Naegleria fowleri* ist ein frei lebender Amöbenflagellat. Er lebt typischerweise in Süßwasser und kann Badende infizieren. Der Parasit dringt über die Nase und die Riechnerven in das Gehirn vor und verursacht eine perakute Meningoenzephalitis. Die Enzephalitisfälle durch Acanthamoeben oder Naeglerien sind extrem selten, haben aber bislang eine extrem schlechte Prognose.

Chemotherapeutika bei *E.histolytica*-Infektionen sind derzeit Nitroimidazole, in erster Linie Metronidazol.

*E.histolytica* besitzt keine oxidative Phosphorylierung sondern gewinnt seine Energie durch Glykolyse. Bei der Oxidation des Pyruvats zu Acetyl-CoA entsteht in der Amöbe reduziertes Ferredoxin, das in der Lage ist, das Nitroimidazol zu einem Nitrosoimidazol zu reduzieren. Durch diese aggressive Substanz werden die Biomoleküle der Amöbe geschädigt. Der Mensch besitzt kein solch starkes reduzierendes Agens und wandelt das Metronidazol nicht in die giftigere Nitrosoimidazolform um. Bisher gibt es noch keine gesicherten Berichte über die Verbreitung Metronidazol - resistenter *E. histolytica* Stämme. Dennoch wird immer wieder von Fällen berichtet, in denen die Metronidazol-Therapie versagt haben soll, und im Labor konnte man schon teilresistente Stämme generieren. Im Falle einer möglichen Resistenzausbildung wäre es sehr wichtig, neue Substanzklassen mit Wirksamkeit gegen *E.histolytica* zu besitzen, da es zur Zeit keine zufrieden stellende Alternative zu den Nitroimidazolen gibt.

Im Gegensatz zu *E. histolytica* besitzen Acanthamoeben und Naeglerien Mitochondrien und können aerob leben. Sie reduzieren Nitroimidazole nicht und deshalb sind diese Verbindungen vollkommen wirkungslos. Acanthamoeben sollen gegen Rifampicin und Paromomycin, Naeglerien gegen Amphotericin B empfindlich sein, dennoch ist erst in wenigen Einzelfällen eine Heilung von Enzephalitiden durch frei lebende Amöben gelungen.

In der DE-Anmeldung P 41 32 344.0-41 sind Verfahren zur Herstellung eines Arzneimittels offenbart, welches zur oralen oder topischen Verabreichung bei der Behandlung von Protozoenerkrankungen, insbesondere der Leishmaniose, geeignet ist und das als Wirkstoff eine oder mehrere Verbindungen der allgemeinen Formel: 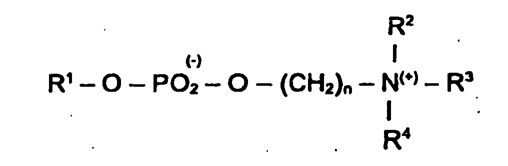 worin R¹ ein gesättigter oder ungesättigter Kohlenwasserstoffrest mit 12 bis 20 C-Atomen ist,
R², R³ und R⁴ jeweils unabhängig H, eine C₁-C₅-Alkylgruppe, eine C₃-C₆-Cycloalkylgruppe oder eine C₁-C₅-Hydroxyalkylgruppe sind, wobei zwei von R², R³ und R⁴ miteinander eine C₂-C₅-Alkylengruppe bilden können, die gegebenenfalls mit einer -O-, -S- oder NR⁵-Gruppe substituiert sein kann, worin R⁵ H, eine C₁-C₅-Alkylgruppe, eine C₃-C₆-Cycloalkylgruppe oder C₁-C₅-Hydroxyalkylgruppe ist, enthält.

Verbindungen dieser allgemeinen Formel zeigten insbesondere bei oraler oder topischer Applikation eine sehr viel höhere Aktivität als Stibogluconat-Natrium. Allerdings traten bei höheren Dosierungen häufig Nebenwirkungen auf wie z.B. Reizung des Magen-Darm-Trakts, die in Versuchstieren zu Appetitlosigkeit und erheblichem Gewichtsverlust führten - in Ratten wurden mehr als 25 % Reduktion des Körpergewichts beobachtet.

Ein weiterer Nachteil der oben genannten Verbindungen besteht darin, dass bisher die intravenöse Gabe von Alkylphosphocholinen mit Kettenlängen von über 21C-Atomen wegen ihrer geringen Wasserlöslichkeit und von Alkylphosphocholinen mit Kettenlängen von 21 oder weniger C-Atomen wegen hämolytischer Wirkungen nicht möglich war. In der Vergangenheit wurden Alkylphosphocholin-haltige Mittel zur intravenösen Verabreichung in Liposomen verpackt. Die Liposomen bestanden aus Hexadecylphosphocholin, Cholesterin und Phosphatidylglycerin oder aus Hexadecylphosphocholin, Cholesterin und Phosphatidylpolyethylenglykolen. Die Herstellung dieser Liposomen ist allerdings sehr aufwendig und teuer, da sie Hochdruckpressen oder ähnliche Verfahren erfordert, und zudem hat das fertige Produkt den Nachteil, dass es nur sehr schwer steril filtrierbar ist.
a) WO 01/72289A beschreibt eine Arzneimittelformulierung, welche ein Gemisch ist aus einer Phospholipidverbindung der Formel I: 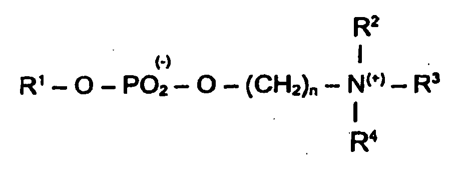 worin R¹ ein gesättigter oder ungesättigter Kohlenwasserstoffrest mit 16 bis 24 C-Atomen ist, R¹, R³ und R⁴ jeweils unabhängig H, eine C₁-C₅-Alkylgruppe, eine C₃-C₆-Cycloalkylgruppe oder eine C₁-C₅-Hydroxyalkylgruppe sind, wobei zwei von R², R³ und R⁴ miteinander eine C₂-C₅-Alkylengruppe bilden können, die gegebenenfalls mit einer -O-, -S- oder NR⁵⁻Gruppe substituiert sein kann, worin R⁵ H, eine C₁-C₅-Alkylgruppe, eine C₃-C₆-Cycloalkylgruppe oder C₁-C₅-Hydroxyalkylgruppe ist, n eine ganze Zahl von 2 bis 6 ist als Wirkstoff zu 30 bis 60 Mol-%,
b) Cholesterin und/oder einem Cholesterinderivat zu 25 bis 65 Mol-% und
c) einem mindestens eine Oleylgruppe enthaltenden Phosphatidylmonoglycerin oder Phosphatidyloligoglycerin zu 5 bis t5 Mol-%, wobei a), b) und c) zusammen 100 Mol-% ergeben und
d) einem wassermischbaren, physiologisch annehmbaren Alkohol mit 2 bis 4 C-Atomen, der gegebenenfalls Wasser enthält, sowie gegebenenfalls übliche pharmazeutische Hilfsstoffe oder/und Wirkstoffe aufweist, wobei die Komponenten als in Wasser dispergierter Komplex vorliegen.

Durch diese Formulierung werden einige der oben genannten Nachteile überwunden.

WO 03/028736A beschreibt eine Arzneimittelzubereitung enthaltend
a) als Wirkstoff 30 bis 60 Mol-% einer Phospholipidverbindung der Formel I: 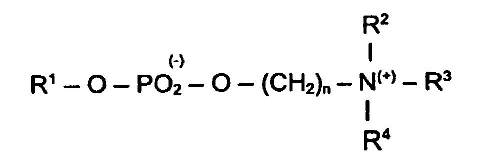 worin R¹ ein gesättigter oder ungesättigter, insbesondere ein einfach oder mehrfach ungesättigter Kohlenwasserstoffrest mit 15 bis 24 C-Atomen, insbesondere mit 16 bis 24C-Atomen ist, welcher gegebenenfalls ein oder mehrere Heteroatome, ausgewählt aus O, N oder S enthalten kann, R², R³ und R⁴ jeweils unabhängig H, eine C₁-C₅-Alkylgruppe, eine C₃-C₆-Cycloalkylgruppe oder eine C₁-C₅-Hydroxyalkylgruppe sind, wobei zwei von R², R³ und R⁴ miteinander eine C₂-C₅-Alkylengruppe bilden können, die gegebenenfalls mit einer -O-, -S- oder NR⁵-Gruppe substituiert sein kann, worin R⁵ H, eine C₁-C₅-Alkylgruppe, eine C₃-C₆-Cycloalkylgruppe oder C₁-C₅-Hydroxyalkylgruppe ist, n eine ganze Zahl von 2 bis 6 ist,
b) Cholesterin zu 10 bis 65 Mol-%, insbesondere zu 25 bis 65 Mol-% und
c) Cholesterylphosphomonoglycerin, Cholesterylphosphooligoglycerin, Alkylphosphoglycerin, Alkylphosphooligoglycerin, Alkylphosphoglycol, Alkylphosphopropandiol-(1,3) oder/und Alkylphosphopropandiol-(1,2) zu 3 bis 30 Mol-%, insbesondere zu 5 bis 15 Mol-%.

Trotzdem besteht, insbesondere auch aufgrund der Häufigkeit des Auftretens und der damit verbundenen Bedeutung der oben genannten Erkrankungen weiterhin ein großer Bedarf an Arzneimittelzubereitungen, die erfolgreich zur Behandlung oder/und Prophylaxe dieser Erkrankungen eingesetzt werden können.

Weiterhin besteht ein großer Bedarf an Formulierungen, die für den Einsatz bei Tieren und insbesondere bei Hunden geeignet sind. Für die Anwendung beim Menschen entwickelte Formulierungen können oftmals bei Tieren und insbesondere bei Hunden nicht eingesetzt werden, da diese wesentlich sensitiver reagieren und oftmals unerwünschte Nebenwirkungen eine Behandlung erschweren oder unmöglich machen.

Wichtig insbesondere für den veterinärmedizinen Bereich sind aber auch die Herstellungs- und die Entwicklungskosten des Arzneimittels.

Eine Aufgabe der vorliegenden Erfindung war deshalb neue Arzneimittelzubereitungen bereitzustellen, die erfolgreich zur Behandlung und/oder Prophylaxe der oben genannten Erkrankungen eingesetzt werden können und die sich insbesondere nicht nur zur Behandlung von Menschen sondern auch zur Behandlung von Tieren und insbesondere von Hunden eignen.

Diese Aufgabe wird erfindungsgemäß gelöst durch eine Arzneimittelzubereitung, enthaltend als Wirkstoff
a) eine Phospholipidverbindung der allgemeinen Formel I (Alkylphosphocholine): 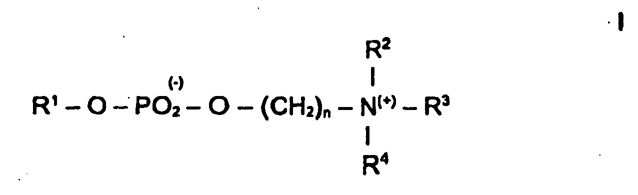 worin R¹ ein gesättigter oder ungesättigter Kohlenwasserstoffrest mit 15 bis 26 C-Atomen ist
   worin R² R³ und R⁴ bei jedem Auftreten jeweils unabhängig H, eine C₁ bis C₆-Alkylgruppe, eine C₃ bis C₈-Cycloalkylgruppe oder eine C₂ bis C₆-Hydroxyalkylgruppe sind, wobei zwei aus R², R³ und R⁴ miteinander eine C₂
   bis C₅-Alkylengruppe bilden können,
   worin n eine ganze Zahl von 2 bis 6 ist,
   oder durch eine Arzneimittelzubereitung, enthaltend als Wirkstoff eine Phospholipidverbindung der allgemeinen Formel II (Alkylphosphocholine mit Ring-Stickstoff): 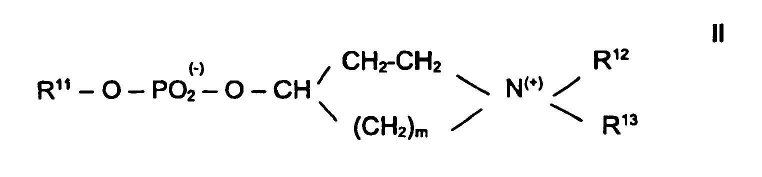 worin R¹¹ ein gesättigter, ungesättigter oder auch mehrfach ungesättigter Kohlenwasserstoffrest mit 15 bis 26 C-Atomen ist,
   worin R¹² und R¹³ bei jedem Auftreten jeweils unabhängig H, eine C₁ bis C₆-Alkylgruppe oder eine C₂ bis C₆-Hydroxyalkylgruppe sind,
   worin m eine ganze Zahl von 1 oder 2 ist,
   oder durch eine Arzneimittelzubereitung, enthaltend als Wirkstoff eine Phospholipidverbindung der allgemeinen Formel III (Alkyl-ethylenglycolphosphocholine) 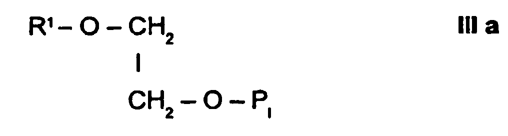 oder 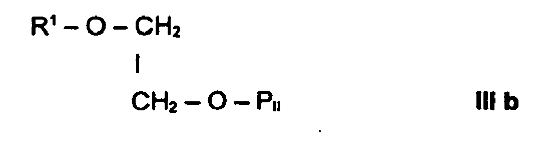 worin P_{I} 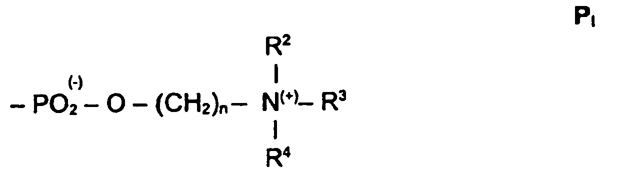 darstellt und P_{II} 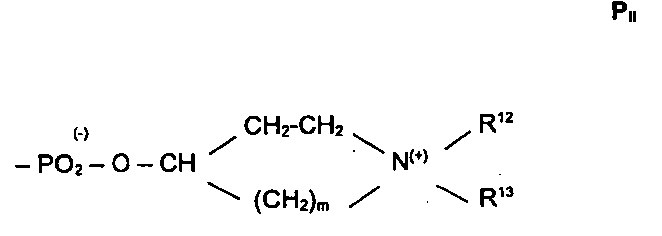 darstellt
   oder durch eine Arzneimittelzubereitung, enthaltend als Wirkstoff eine Phospholipidverbindung der allgemeinen Formel IV (Alkyl-alkandiol-phosphocholine) 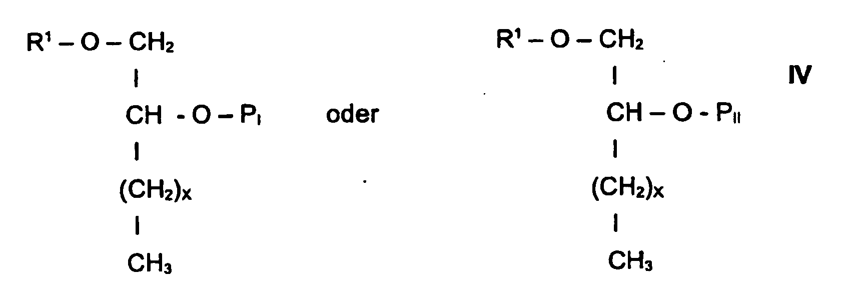 worin die Reste R¹ und P_{I} oder P_{II} auch gegeneinander ausgetauscht werden können,
   worin R¹, P_{I} und P_{II} den obigen Bedeutungen entsprechen,
   worin x eine ganze Zahl von 0 bis 4 ist,
   oder durch eine Arzneimittelzubereitung, enthaltend als Wirkstoff eine Phospholipidverbindung der allgemeinen Formel V (Ether-lysolecithine) 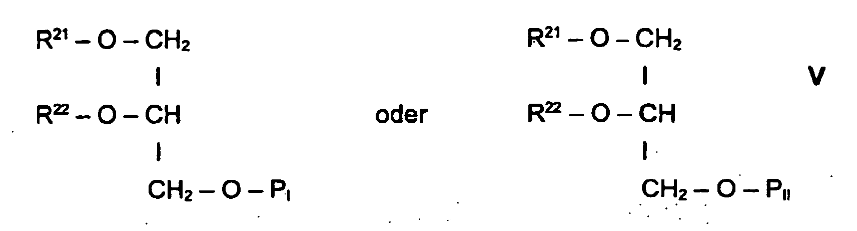 worin R²¹ ein gesättigter oder ein einfach oder mehrfach ungesättigter Kohlenwasserstoffrest mit 15 bis 26 C-Atomen ist,
   worin R²² eine C₁ bis C₆-Alkylgruppe bedeutet,
   worin P_{I} und P_{II} den obigen Bedeutungen entsprechen, wobei die Reste R²¹, R²² und P_{I} bzw. P_{II} beliebig über die Positionen im Glycerinmolekül verteilt werden können
   oder durch eine Arzneimittelzubereitung, enthaltend als Wirkstoff eine Phospholipidverbindung der allgemeinen Formel VI (Alkyl-substituierte Alkandiol-phosphocholine): 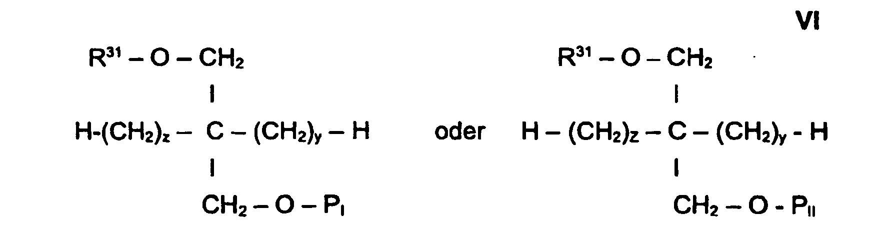 worin R³¹ ein gesättigter oder einfach oder mehrfach ungesättigter Kohlenwasserstoffrest mit 15 bis 26 C-Atomen ist,
   worin y und z unabhängig voneinander eine ganze Zahl von 0 bis 3 sein können, aber y und z nicht beide gleichzeitig 0 sein können,
   worin P_{I} und P_{II} den obigen Bedeutungen entsprechen,
   oder durch eine Arzneimittelformulierung, enthaltend als Wirkstoff eine Phospholipidverbindung der allgemeinen Formel VII (1-O-Alkyl-2-methylgycero-3-phosphocholin) 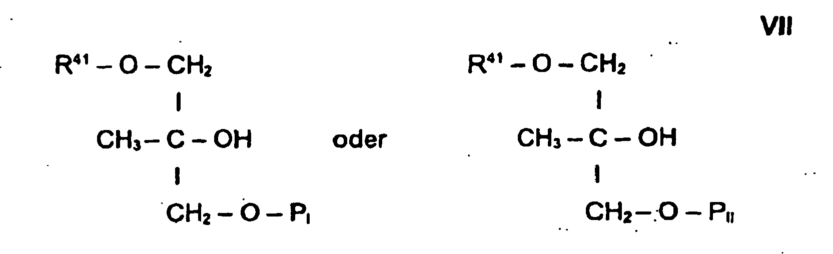 worin R⁴¹ ein gesättigter oder einfach oder mehrfach ungesättigter Kohlenwasserstoffrest mit 15 bis 26 C-Atomen ist,
   worin P_{I} und P_{II} die oben angegebenen Bedeutungen haben
   **dadurch gekennzeichnet,**
   dass die Zusammensetzung weiterhin den Bestandteil
b) umfassend Cholesterin, 7 β-Hydroxycholesterin oder/und ein ß-Sitosterin sowie als Bestandteil
c) einen negativen Ladungsträger, ausgewählt aus
einer Carbonsäure mit 16 bis 36 C-Atomen, die bevorzugt eine gesättigte, einfach ungesättigte und mehrfach ungesättigte Fettsäure ist und besonders bevorzugt cis-Doppelbindungen enthält.

Als Bestandteil c) wird weiterhin ein negativer Ladungsträger offenbart, der ausgewählt ist aus einer Verbindung aus der Naturstoffklasse der Gallensäuren,
oder Fettsäureamiden von Aminosäuren mit Fettsäuren, die gesättigt oder, einfach oder mehrfach ungesättigt sein können,
oder einer Verbindung der allgemeinen Formel VIII (Fettsäureamide von GPE) 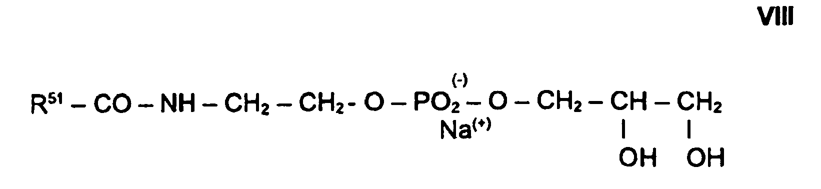 worin R⁵¹ einen Kohlenwasserstoffrest mit 15 bis 25 Kohlenstoffatomen darstellt.

Erfindungsgemäß wurde festgestellt, dass durch gezielte Auswahl bestimmter negativer Ladungsträger Arzneimittelzubereitungen und insbesondere Liposome mit hervorragenden Eigenschaften gewonnen werden können. Als negativer Ladungsträger wird bevorzugt eine Carbonsäure, insbesondere eine Fettsäure, mit 16 bis 36 C-Atomen, mehr bevorzugt mit 16 bis 24 und am meisten bevorzugt mit 16 bis 22 C-Atomen verwendet. Die Carbonsäure kann linear oder verzweigt und gesättigt oder ein- oder mehrfach ungesättigt sein. Bevorzugt sind mehrfach ungesättigte Fettsäuren mit cis-Doppelbindungen. Besonders bevorzugt wird Ölsäure, Linolsäure, Erucasäure oder Retinolsäure eingesetzt. Gallensäuren weisen ebenfalls eine Carboxylgruppe auf. Bevorzugt eingesetzte Vertreter der Gallensäuren sind Cholsäure, Desoxycholsäure, Lithocholsäure, Chenodesoxycholsäure, Cholan-24-säure sowie Ursodesoxycholsäure.

Die erfindungsgemäß als negativer Ladungsträger eingesetzten Carbonsäuren zeichnen sich durch ihre leichte Verfügbarkeit und Kostengünstigkeit aus.

In einer bevorzugten und kostengünstigen Alternative wird eine Kombination von Säuren eingesetzt, welche durch Hydrolyse aus Olivenöl gewonnen wurden, das ca. 85 % Ölsäure, 10 % Linolsäure und restliche 5 % an weiteren Fettsäuren enthält. Für die Preisgestaltung der Formulierung spielen allerdings die Fettsäuren eine untergeordnete Rolle, da Fettsäuren < 10 % der Gesamtformulierung ausmachen. Als Ausgangsprodukt für die Synthese des Wirkstoffs Oleylphosphocholin ist Olivenöl allerdings eine interessante Alternative zu der Verwendung von reiner Ölsäure als Ausgangsprodukt.
Besonders gute Ergebnisse werden erzielt, wenn eine im Wesentlichen deprotonierte Carbonsäure eingesetzt wird, beispielsweise ein Carbonsäure, die zu mehr als 50 %, mehr bevorzugt zu mehr als 70 %, noch mehr bevorzugt zu mehr als 90 % und am meisten bevorzugt zu mehr als 95 % deprotoniert ist. Die Deprotonierung kann durch Einstellung des pH-Werts, beispielsweise durch Zugabe von Lauge zu Carbonsäurelösungen gebildet werden. Bei Zugabe von Natronlauge zu Carbonsäuren, werden beispielsweise die Natriumsalze der Carbonsäuren gebildet.

Bei Verwendung von Carbonsäuren mit einem pK-Wert von ungefähr 5, wird zusätzlich eine vorteilhafte Pufferwirkung erhalten. Da zahlreiche Carbonsäuren, wie z.B. Ölsäure zudem hitzestabil sind, können erfindungsgemäße hitzesterilisierbare Arzneimittelzubereitungen und insbesondere hitzesterilisierbare Liposome erhalten werden.

Als negativer Ladungsträger werden weiterhin bevorzugt Fettsäureamide von Aminosäuren eingesetzt. Auch hier ist die deprotonierbare Gruppe eine Carboxylgruppe. Als Aminosäure werden bevorzugt die natürlichen Aminosäuren wie z.B. Glycin, Sarkosin, Alanin, Serin, usw., eingesetzt. Der Fettsäureanteil der Fettsäureamide stammt bevorzugt von ungesättigten oder einfach oder mehrfach ungesättigten, mehr bevorzugt eine cis-Doppelbindung aufweisenden Fettsäuren (C₁₆ bis C₃₆).

Weiterhin wurde festgestellt, dass weitere negative Ladungsträger, welche zur Bildung von Liposomen geeignet sind, ausgehend von Glycerophosphoethanolamin (G-PE) erhalten werden können, welches ein Nebenprodukt bei der Sojaverarbeitung ist. An das Glycerophosphoethanolamin kann mittels einer Amidbindung eine Fettsäure eingeführt werden. Durch die Blockierung der sonst positiv geladenen Aminogruppe wird somit ein insgesamt negativ geladenes Molekül, welches als negativer Ladungsträger geeignet ist, erhalten. Ein wesentlicher Unterschied zu den vorher beschriebenen negativen Ladungsträgern, die alle auf der Deprotonierung einer Carboxylgruppe (pk 5 bis 6) beruhen, liegt hier in der Verwendung einer Phosphatgruppe als negativer Ladungsträger mit pK-Werten um 2.

Der Rest R⁵¹ in Verbindungen der Formel VIII ist ein gesättigter oder ein einfach oder mehrfach ungesättigter Rest, welcher bevorzugt cis-Doppelbindungen enthält.

Als Wirkstoff sind Verbindungen der Formel I bevorzugt, in denen R¹ ein einfach ungesättigter Kohlenwasserstoff mit 15 bis 26 C-Atomen ist wie z.B. Oleyl oder Erucyl mit jeweils einer cis-Doppelbindung mit jeweils n = 2 oder n = 3, aber auch mit dem gesättigten Rest Hexadecyl. Für Verbindungen der Formel II gilt ebenso eine bevorzugte Verwendung von R¹ als einfach ungesättigtem Kohlenwasserstoff mit 15 bis 26 C-Atomen wie z.B. Oleyl oder Erucyl mit jeweils einer cis-Doppelbindung mit jeweils n = 2 oder n = 3, aber auch mit dem gesättigten Rest Octadecyl.

a), b) und c) ergeben zusammen bevorzugt 100 Mol-%. Der Formulierung können aber weiterhin ein wassermischbarer, physiologisch annehmbarer Alkohol mit 2 bis 4 C-Atomen, der gegebenenfalls Wasser enthält, sowie gegebenenfalls übliche pharmazeutische Hilfsstoffe oder/und Wirkstoffe zugegeben sein. Die Komponenten liegen bevorzugt als in Wasser dispergierte Komplexe in Form von Liposomen vor, die üblicherweise einen Durchmesser von 70 bis 150 nm aufweisen, also noch problemlos steril filtriert werden können. Von noch größerer Bedeutung ist jedoch, dass die Formulierungen hitzesterilisiert und bei 20 °C aufbewahrt werden können. Diese so genannten Liposomen bilden sich im Wasser durch milde Ultraschallbehandlung. Es werden zur Herstellung also keine Hochdruckhomogenisatoren benötigt.

Es wurde überraschenderweise festgestellt, dass die Verpackung der Wirkstoffe in den erfindungsgemäßen Liposomen erhebliche Vorteile gegenüber den freien Wirkstoffen liefert. Durch die Verpackung in den Liposomen kann insbesondere eine Erhöhung der gewünschten Wirksamkeit und gleichzeitig eine Verringerung unerwünschter Nebenwirkungen erzielt werden. So ist die hämolytische Aktivität bei Verabreichung in liposomaler Form deutlich herabgesetzt, wie im Hämolysetest gezeigt werden konnte. Das in den Liposomen mit eingeschlossene Cholesterin, 7-β-Hydroxy-Cholesterin oder β-Sitosterin unterdrückt die hämolytische Aktivität der Alkylphosphocholine, also der Wirkstoffverbindungen. Dadurch können die Wirkstoffe in viel höheren Konzentrationen eingesetzt und appliziert werden. Während die freien Wirkstoffe schon bei Konzentrationen um 10⁻⁴ M gewebereizend sind und zu Geschwüren führen, können in liposomaler Form Konzentrationen dieser Wirkstoffe von > 60 mM eingesetzt werden. Dabei ist jede Applikationsform möglich, wie z.B. intravenös, intramuskulär, subkutan, u.s.w. bis zur oralen oder topischen Anwendung.

Auffallend ist, dass die hier vorgestellten liposomalen Formulierungen auch bei oraler Gabe deutliche Vorteile gegenüber den freien Wirkstoffen, Alkylphosphocholine in physiologischer Kochsalzlösung, besitzen. Bei höheren Konzentrationen der freien Alkylphosphocholine kommt es in Ratten als Versuchstieren zu massiven Reizungen des Magen-Darm-Trakts verbunden mit Appetitlosigkeit, die zu enormem Gewichtsverlust führen, bis zu 30 % in einem Zeitraum von 3 Wochen. Versuche mit entsprechenden Mengen an Alkylphosphocholinen in Form von liposomaler Formulierungen sind völlig problemlos. Die Tiere zeigen keine Auffälligkeiten, Gewichtsverlust wird nicht beobachtet. Die orale Therapie mit liposomalen Alkylphosphocholinen ist also der Therapie mit den freien Wirkstoffen deutlich überlegen.

Im Stand der Technik wurden oftmals Hilfsstoffe verwendet, die pharmakologisch neu und deren Toxizität nicht bekannt waren - eine Erschwernis, die neben den damit verbundenen Unkosten, die Unbedenklichkeit dieser Substanzen abzuklären, auch zu erheblichen zeitlichen Verzögerungen geführt hätte. Weiter war die Lagerstabilität der hier vorgesehenen liposomalen Dispersionen bei 20 °C von großer Bedeutung, da diese Arzneimittel häufig zu südlichen Ländern zum Einsatz kommen. Die Formulierung sollte nach Möglichkeit auch hitzesterilisierbar sein, um eine Freigabe des Arzneimittels über einen Zeitraum von 3 Jahren zu erhalten. Alle diese Voraussetzungen, ja fast Bedingungen für einen Einsatz im veterinärmedizinen Bereich, konnten für die hier vorgestellten liposomalen Formulierungen eingehalten werden.

Die Erfindung beruht darauf, dass die unter den Formeln I bis VII genannten Wirkstoffe einfach als integrale Bestandteile von Liposomen eingebracht werden - gemeinsam mit Cholesterin, 7-β-Hydroxy-Cholesterin oder β-Sitosterin und einem negativen Ladungsträger. Die Wirkstoffe können kostengünstig hergestellt werden. Cholesterin und β-Sitosterol können preisgünstig eingekauft werden und die meisten negativen Ladungsträger ebenso.

Von großer Bedeutung ist auch die einfache und kostengünstige Herstellung der Liposomen, ihre Hitzesterilisierbarkeit und Lagerfähigkeit bei 20 °C. Damit stehen liposomale Formulierungen von Wirkstoffen zur Verfügung, die bei den genannten lebensbedrohenden Erkrankungen weitgehend nebenwirkungsfrei eingesetzt werden können.

Die erfindungsgemäßen Arzneimittelzubereitungen können als Wirkstoff eine Phospholipid-, insbesondere eine Alkylphosphocholinverbindung enthalten.

In der Komponente a), dem Phospholipid bzw. Alkylphosphocholin der Formel I, kann der Kohlenwasserstoffrest R¹ 16 bis 26 C-Atome enthalten, bevorzugt sind insbesondere 16 bis 24 und stärker bevorzugt 18 bis 22 C-Atome. Besonders bevorzugt ist R¹ ein Alkylrest oder ein einfach oder mehrfach ungesättigter Alkenylrest und insbesondere ein Hexadecyl-, Heptadecyl-, Octadecyl-, Nonadecyl-, Eicosyl-, Hexadecenyl-, Heptadecenyl-, Octadecenyl-(Oleyl), Nonadecenyl-, Eicosenyl-, Elaidyl-, Eicosenyl-cis-(w-9)-, Heneicosyl-, Heneicosenyl-, Docosyl-, Docosenyl-, Linoleyl-, Linolenyl-, Erucyl- oder Tetracosadienyllrest. Der Kohlenwasserstoffrest kann gesättigt oder einfach oder mehrfach ungesättigt, insbesondere aber einfach oder zweifach ungesättigt sein, wobei die Doppelbindung(en) der ungesättigten Reste ganz bevorzugt cis sind. Ist mehr als eine cis-Doppelbindung vorhanden, so liegen diese bevorzugt nicht in Konjugation vor. Der Kohlenwasserstoffrest kann verzweigt oder linear sein, ist aber bevorzugt linear.

Für die Behandlung von Leishmaniose sind Verbindungen mit cis-Doppelbindungen wie z.B. Nonadecenyl, Eicosenyl, Heneicosenyl und Oleyl besonders bevorzugt. Am meisten bevorzugt ist aber die Verbindung der Formel I mit R¹ = Oleyl und n = 2, also Oleylphosphocholin.

Für die Behandlung von Krebs sind die Verbindungen mit cis-Doppelbindung wie z.B. Oleyl und Erucyl besonderes bevorzugt. Am meisten bevorzugt sind zwei Verbindungen der Formel I, a) mit R¹ = Erucyl und n = 2, also Erucylphosphocholin und b) mit R¹ = Erucyl und n = 3, also Erucyl-phospho-(N.N.N)-trimethyl-propylammonium.
Verbindungen mit einem ungesättigten Rest R¹ haben den Vorteil, dass sie eine große therapeutische Breite und zugleich eine sehr geringe Toxizität aufweisen, wie zum Beispiel Oleylphosphocholin, Erucylphosphocholin und Erucylphospho-(N.N.N.-trimethyl)-propylammonium.

In Formel I sind R² R³ und R⁴ bevorzugt jeweils Methyl. Beispiele für andere geeignete Reste sind Ethyl-, Propyl-, Butyl- und Pentylreste, Cyclopropyl-, Cyclobutyl-, Cyclopentyl- und Cyclohexylreste, Hydroxyethyl- und Hydroxypropylreste. Zwei der Reste R² R³ und R⁴ können beispielsweise eine Pyrrolidin-, eine Piperidin- oder eine Morpholingruppe bilden. Vorzugsweise ist mindestens einer der Reste R², R³ und R⁴ von Wasserstoff verschieden, besonders bevorzugt sind alle drei Reste von Wasserstoff verschieden.

Der polare Bestandteil der Verbindungen der Formel I besteht bevorzugt aus Phosphocholin (PC), d.h. n ist besonders bevorzugt gleich 2 - n kann bevorzugt auch 3 oder 4 sein. Überraschenderweise ergab sich besonders bei Verbindungen mit n gleich 3 eine stimulierende Wirkung auf die Leukopoese.

Als Bestandteil a) geeignete und verwendbare Alkylphosphocholine sind z.B. die in EP 0 507 337 oder WP 00/08031 beschriebenen Verbindungen.

Bei den Phospholipidverbindungen, insbesondere bei solchen mit kurzen Kohlenwasserstoffketten im Rest R¹ tritt bei herkömmlichen Formulierungen oft ein schädlicher hämolytischer Effekt auf. Dieser wird durch die erfindungsgemäße Kombination wesentlich gemindert. Bei kurzkettigen Resten R¹ mit Kohlenwasserstoffresten mit 15 bis 21 C-Atomen sind daher Cholesterin bzw. Cholesterinderivate bzw. ß-Sitosterine im oberen angegebenen Merigenbereich bevorzugt. Es ist somit bevorzugt, einen kleinen Überschuss an Cholesterin bzw. dessen Derivat im Komplex bzw. Liposom zu haben, sodass das Molverhältnis zwischen Verbindung der Formel I und Cholesterin/-derivat bevorzugt 1 **:** 1-1,2 ist. Für erfindungsgemäße Zusammensetzungen, in denen der als Bestandteil a) eingesetzte Wirkstoff einen Rest R mit ≤ 21 C-Atomen aufweist, beträgt das Mischungsverhältnis bevorzugt 30 bis 45 Mol-% Bestandteil a), 30 bis 60 Mol-% Bestandteil b) (Cholesterin) und 3 bis 30 Mol-% Bestandteil c) d.h. negative Ladungsträger wie Fettsäuren usw.

Bei Verbindungen mit einem Rest R¹ längeren Kohlenwasserstoffketten mit 22 bis 26, insbesondere bis 24 Kohlenstoffatomen besteht weniger das Problem der Hämolyse als das einer geringeren Wasserlöslichkeit. Aus diesem Grund reicht hier ein Molverhältnis von Phospholipidverbindung: Cholesterin/-derivat bevorzugt von 1:0,5-1 aus.

Für erfindungsgemäße Zusammensetzungen, in denen als Wirkstoff der Komponente a) eine Verbindung mit R¹ ≥ 22 C-Atomen eingesetzt wird, beträgt das Mischungsverhältnis der einzelnen Komponenten bevorzugt 30 bis 55 Mol-% Komponente a), 10 bis 40 Mol-% Komponente b) (Cholesterin) und 3 bis 30 Mol-% Komponente c) also negative Ladungsträger wie Fettsäuren usw.

Die Bedingungen für eine gute antineoplastische Wirksamkeit, insbesondere Halbwertszeiten in Organen und Geweben von 60 bis 100 Stunden können durch die oben angegebenen Wirkstoffe erzielt werden.

Ganz allgemein kann gesagt werden, dass die Verbindungen der Formeln I bis VII sehr ähnliche physikalische Eigenschaften besitzen und mit Lysolecithinen verglichen werden können. Diesen Molekülen ist gemeinsam eine lange Alkylkette, die direkt mit Phosphocholin (Formel P_{I}) oder einem Analogon (Formel P_{II}) verknüpft sein kann, wie im Falle der Alkylphosphocholine. Die Verknüpfung kann aber auch über eine Diolbrücke erfolgen, z.B. in Formel III über Ethylenglykol, in Formel IV über Alkandiol-(1.2), in Formel V über Glycerin, in Formel VI über Propandiol-(1.3), in Formel VII über 2-Methyl-glycerin.

Physikalisch in den hämolytischen und cytolytischen Eigenschaften sind diese Moleküle Lysolecithinen als körpereigenen Substanzen sehr ähnlich, auch die kritischen Mizellkonzentrationen sind vergleichbar bei gleicher Alkylkettenlänge. Biologisch jedoch gibt es dramatische Unterschiede. Die Halbwertzeit von Lysolecithinen in biologischen Zellen und Organen ist kurz, < 1 Min. Lysolecithine werden rasch metabolisiert, d.h. durch Acylierung über Acyltransferasen in Lecithine umgewandelt. Diese Reacylierung ist bei den Molekülen der Formel I bis VI nicht möglich - deshalb unterbunden, weil die freie sekundäre Hydroxylgruppe im Lysolecithin fehlt oder bereits blockiert ist (siehe Formel V). Eine besondere Situation finden wir in Formel VII. Dort ist zwar eine Hydroxylfunktion in Position 2 vorhanden. Es handelt sich aber um eine tertiäre Hydroxylgruppe, die durch Acryltransferasen nicht acyliert werden kann. Im Unterschied zu Lysolecithinen haben deshalb die Verbindungen der Formeln I bis VII lange biologische Halbwertszeiten von 60 bis 100 Stunden.

Die erfindungsgemäßen Arzneimittelzubereitungen können als Wirkstoff neben den zuvor ausführlich diskutierten Alkylphosphocholinen der Formel I auch Verbindungen der Formeln II bis VII enthalten. Die hier zuvor diskutierten bevorzugten Bedeutungen für R¹ gelten in gleicher Weise für die Reste R¹¹, R²¹, R³¹ und R⁴¹. Die schon erwähnten großen Ähnlichkeiten in den physikalischen Eigenschaften gehen sogar soweit, dass auch die Schutzfunktion des Cholesterins, des β-Hydroxy-Cholesterins und des β-Sitosterins für alle der genannten Substanzen unter den Formeln II bis VII erhalten bleibt, insbesondere die Schutzfunktion gegen Hämolyse und Cytolyse. Die für die Alkylphosphocholine der Formel I detailliert diskutierten Reste stellen auch die entsprechenden bevorzugten Reste in den Substanzen der Formeln II bis VII dar.

In den Alkylphosphocholin analogen Verbindungen der Formeln II bis VII kann der Kohlenwasserstoffrest R¹¹, R²¹ R³¹ bzw. R⁴¹ 16 bis 26 C-Atome enthalten, bevorzugt sind insbesondere 16 bis 22, stärker bevorzugt 18 bis 22 C-Atome. Besonders bevorzugt ist mindestens eine cis-Doppelbindung im Molekül vorhanden. Besonders bevorzugt ist R¹¹, R²¹ R³¹ oder R⁴¹ ein Alkylrest, ein Alkenylrest, ein Alkadienylrest oder ein Alkaltrienylrest und insbesondere ein Hexadecyl-, Heptadecyl-, Octadecyl-, Nonadecyl-, Eicosyl-, Hexadecenyl-, Heptadecenyl-, Octadecenyl-(Oleyl), Linolyl-, Linolenyl-, Nonadecenyl-, Eicosenyl-, Elaidyl-, Eicosenyl-cis-(w-9)-, Heneicosyl-, Heneicosenyl-, Docosyl- oder Docosenylrest. Der Kohlenwasserstoffrest kann gesättigt oder einfach oder mehrfach ungesättigt sein, insbesondere aber einfach oder zweifach ungesättigt sein, wobei die Doppelbindung(en) der ungesättigten Reste bevorzugt cis sind. Ist mehr als eine cis-Doppelbindung vorhanden, liegen diese bevorzugt nicht in Konjugation vor. Der Kohlenwasserstoffrest kann verzweigt oder linear sein und ist bevorzugt linear.
Für die Behandlung von Leishmaniose sind Verbindungen mit cis-Doppelbindungen wie z.B. Nonadecenyl, Eicosenyl, Heneicosenyl und Oleyl besonders bevorzugt. Am meisten bevorzugt sind die Verbindungen der Formeln II bis VII mit R¹¹ R²¹, R³¹ bzw. R⁴¹=Oleyl und n = 2, d.h. für Formel II: Oleyl-ethylenglykol-phospho-(N.N-dimethyl)-pyrrolidinium; für Formel III: Oleyl-ethylenglycol-phosphocholin (Version P_{I}) oder Oleyl-ethylenglycolphospho-(N.N-dimethyl)-pyrrolidinium (Version P_{II}): für Formel IV: 1-Oleylpropandiol-(1.2)-phosphocholin (Version P_{I}) oder 1-Oleyl-propandiol-(1.2)-phospho-(N.N-dimethyl)-pyrrolidinium (Version P_{II}) ; für Formel V: 1-Oleyl-2-methyl-glycero-3-phosphocholin (Version P_{I}) oder 1-Oleyl-2-methyl-glycero-3-phospo-(N.N-dimethyl)-pyrrolidinium (Version P_{II}) einschließlich aller Stereo- und Konfigurationsisomeren; für Formel VI: Oleyl-2.2-dimethylpropandiol-(1.3)-phosphocholin (Version P_{I}) oder Oleyl-2.2-dimethylpropandiol-(1.3)-phospho-(N.N-dimethyl)-pyrrolidinium (Version P_{II}); für Formel VII: 1-Oleyl-2-methyl-propantriol-phosphocholin (Version P_{I}) oder 1-Oleyl-2-methyl-propantriol-phospho-(N.N-dimethyl)-pyrrolidinium.

Für die Behandlung von Krebs sind die entsprechenden Verbindungen mit Oleyl und Erucyl besonders bevorzugt, wieder mit jeweils einer cis-Doppelbindung. Am meisten bevorzugt sind jedoch alle Verbindungen der Formeln II bis VII, die in R¹¹ R²¹, R³¹ bzw. R⁴¹ einen Erucylrest tragen, z.B. für Formel II: Erucyl-phospho-(N.N-trimethyl)-pyrrolidinum; für Formel III: Erucyl-ethylenglycol-phosphocholin (Version P_{I}, n = 2) oder Erucyl-ethylenglycol-phospho-(N.N.N-trimethyl)-propylammonium (Version P_{I}, n = 3); oder für Formel IV: Erucyl-propandiol-(1.2)-phosphocholin (Version P_{I} n = 2) oder Erucyl-propandiol-(1.2)-phospho-(N.N.N-trimethyl)-propylammonium (Version P_{I}, n = 3), oder für Formel V: 1-Erucyl-2-methyl-glycero-3-phosphocholin (Version P_{I}, n = 2) oder 1-Erucyl-2-phospho-(N.N.N-trimethyl)-propylammonium (Version P_{I}, n =3); oder für Formel VI: Erucyl-2.2-dimethyl-propandiol-(1.3)-phosphocholin (Version P_{I}, n = 2) oder Erucyl-2.2-dimethyl-propandiol-1.3-phospho-(N.N.N-trimethyl)-propylammonium (Version P_{I}, n = 3); oder für Formel VII: Erucyl-2-methyl-propantriolphosphocholin (Version P_{I}, n = 2) oder Erucyl-2-methyl-propantriol-phospho-(N.N.N-trimethyl)-propyl-ammonium.

Besonders bevorzugt sind Hexadecyl, Octadecyl und Oleyl. Am meisten bevorzugt ist eine Verbindung der Formel I mit R¹ = Oleyl-, insbesondere cis-Oleyl-Rest. Weitere bevorzugte Reste R¹ sind Linoleyl-, Linolenyl- sowie Erucylreste.

Verbindungen mit einem ungesättigten Rest R¹¹, R²¹, R³¹ bzw. R⁴¹ haben den Vorteil, dass sie eine große therapeutische Breite und zugleich eine sehr geringe Toxizität aufweisen. Dadurch sind, im Vergleich zu Verbindungen mit gesättigten Resten, höhere Dosierungen möglich.

R², R³ und R⁴ sind bevorzugt Methyl, Ethyl oder Propyl.

In der Ausführung mit P_{I} und R², R³ und R⁴ sind diese Reste bevorzugt jeweils Methyl. Beispiele für andere geeignete Reste sind Ethyl-, Propyl-, Butyl- und Pentylreste, Cyclopropyl-, Cyclobutyl-, Cyclopentyl- und Cyclohexylreste, Hydroxymethyl-, Hydroethyl- und Hydroxypropylreste. Zwei der Reste R², R³ und R⁴ können beispielsweise eine Pyrrolidin-, eine Piperidin- oder eine Morpholingruppe bilden.

Die Menge an Wirkstoff der Formeln I bis VII in den erfindungsgemäßen Arzneimittelzubereitungen beträgt 10 bis 60 Mol-%, bevorzugt mindestens 20 Mol-%, insbesondere mindestens 25 Mol-%, mehr bevorzugt mindestens 40 Mol-% bis zu bevorzugt 50 Mol-%, insbesondere bis zu 45 Mol-%.

Als weiteren Bestandteil enthalten die erfindungsgemäßen Liposomen Cholesterin oder ß-Sitosterol. Unter Cholesterin, wie hierin verwendet, werden Cholesterin selbst sowie Cholesterinderivate verstanden, z.B. 7-β-Hydroxycholesterin.

Anstelle oder neben Cholesterin kann als Komponente b) auch ein β-Sitosterin enthalten sein. ß-Sitosterine sind Pflanzensteroide, die in gewissen Teilbereichen Cholesterin sehr ähnlich sind und üblicherweise ein oder zwei Methylgruppen mehr als Cholesterin enthalten. Auf diese Weise ist es möglich, cholesterinfreie Liposomen herzustellen. Solche Arzneimittelzubereitungen sind insbesondere bei Patienten mit Problemen des Cholesterinspiegels vorteilhaft, da sie keine Erhöhung des Cholesterinspiegels bedingen und zudem ausschließlich aus pflanzlichen Ausgangsmaterialien herstellbar sind. β-Sitosterol hat sogar eine gewisse Bedeutung zur Absenkung des Cholesterinspiegels erlangt.
Die Menge an Cholesterin bzw. β-Sitosterin beträgt 10 is 60 Mol-%, bevorzugt 25 bis 50 Mol-%, insbesondere 30 bis 40 Mol-%.

Die Komponente c) der erfindungsgemäßen Liposome ist ein negativer Ladungsträger, wie oben diskutiert. R^{a} und R¹⁰ stellen bevorzugt einen Kohlenwasserstoffrest mit 16 bis 22 C-Atomen dar. Bevorzugt beträgt die Menge an Bestandteil c) 5 bis 15 Mol-%, insbesondere 7 bis 10 Mol-%.

In einer bevorzugten Ausführungsform enthält die erfindungsgemäße Arzneimittelzubereitung als Wirkstoff a) eine Verbindung, worin R¹, R¹¹, R²¹, R³¹ bzw. R⁴¹ ein gesättigter oder ungesättigter Kohlenwasserstoffrest mit 16 bis 26 C-Atomen ist und als Bestandteil c) als negativen Ladungsträger eine Carbonsäure mit 16 bis 36 C-Atomen. Besonders bevorzugt werden diese beiden Komponenten so gewählt, dass der Rest R¹, R¹¹, R²¹, R³¹ bzw. R⁴¹ gerade dem Carbonsäurerest des Bestandteils c) entspricht, beispielsweise wird für R¹ etc. ein Oleylrest verwendet und als negativer Ladungsträger Ölsäure.

Überraschenderweise wurde festgestellt, dass unter Verwendung der vorstehend genannten negativen Ladungsträger Liposome erhalten werden können, die hitzestabil sind und somit hitzesterilisiert werden können. Dies stellt einen beträchtlichen Vorteil gegenüber anderen Liposomformulierungen dar, insbesondere im Hinblick auf eine mögliche intravenöse oder subkutane Verabreichung der Liposome. Bevorzugt werden deshalb solche Liposome nach ihrer Bildung und vor einer Verabreichung hitzesterilisiert.

Die erfindungsgemäßen Arzneimittelzubereitungen, insbesondere auch in Form von Liposomen weisen zudem eine hohe Stabilität und lange Lagerfähigkeit auf. So sind in den erfindungsgemäßen Formulierungen alle Bestandteile einschließlich des negativen Ladungsträgers in Wasser stabil. Auch bei extremen pH-Wert-Bereichen von pH 1 bis pH 11 tritt keine merkliche Zersetzung auf. Zudem weisen die Formulierungen eine hohe Stabilität gegenüber Sauerstoff auf. Die Lagerstabilität auch bei hohen Umgebungstemperaturen, welche die erfindungsgemäßen Arzneimittelzubereitungen aufweisen, ist insbesondere für den Einsatz in warmen Ländern ein wesentlicher Vorteil, wobei die hierin diskutierten Erkrankungen oftmals gerade in warmen, südlichen Ländern ein großes Problem darstellen. Die hohe Lagerstabilität kommt zudem Veterinären sehr entgegen, da diese erforderliche Medikamente oft über längere Zeiträume lagern müssen.

Vorzugsweise enthält die Arzneimittelformulierung die Bestandteile in einer solchen Menge, dass sie insgesamt eine negative Überschussladung aufweist. Dies ist insbesondere bei der Verwendung von Wirkstoffen mit längeren Kohlenwasserstoffketten vorteilhaft. Das Problem einer schlechteren Wasserlöslichkeit bei Verbindungen mit längeren Ketten, wie z.B. C₂₂ , spielt insbesondere bei einer intravenösen Verabreichung eine Rolle, dagegen ist eine orale Applikation nicht sinnvoll, da < 10 % resorbiert werden.

Das molare Verhältnis der einzelnen Bestandteile der erfindungsgemäßen Liposome kann variieren, sodass z.B. Wirkstoffe der Formeln I bis VII in einem leichten Unterschuss vorhanden sind. Bevorzugt ist jedoch im Allgemeinen ein Verhältnis, das nicht zu weit von 1 : 1 abweicht, z.B. ein molares Verhältnis Wirkstoff der Formeln I bis VII zu Cholesterin bzw. β-Sitosterol von 1 : 1,2 bis 1 : 1. Das Cholesterin bzw. ß-Sitosterol ist bevorzugt zu 45 bis 55 Mol-%, im Liposom vorhanden.

Der aus den Bestandteilen a), b) und c) gebildete liposomenartige Komplex ist problemlos durch Membranen mit Porendurchmesser 0,8 µ, 0,45 µ und sogar 0,2 µ sterilfiltrierbar. Dies stellt einen beträchtlichen Vorteil gegenüber herkömmlichen Liposomen dar, die nicht einfach steril zu filtrieren sind. Von größter Bedeutung ist jedoch, dass die Liposomen hitzesterilisiert werden können. Außerdem hat sich überraschenderweise herausgestellt, dass erfindungsgemäße Liposome extrem lagerstabil sind.

Die Komponenten a, b und c stellen zusammen bevorzugt 100 % der erfindungsgemäßen Liposomen dar. Es ist aber auch möglich Liposome zu bilden, welche weitere Hüllbestandteile oder/und eingekapselte Substanzen enthalten. Bevorzugt sind Liposome, die weitere Wirkstoffe in eingekapselter Form umfassen. Zusätzliche Wirkstoffe, welche vorteilhaft in den erfindungsgemäßen Liposomen enthalten sein können sind z.B. Oxytetracyclin, Doxycyclin oder Minocyclin, welche bakterizid wirksam sind; Amphotericin B oder Griseofulvin, welche fungizid wirksam sind sowie Cyclosporin, welches immunsupprimierend wirkt - sowie Arthemeter und verwandte Substanzen, die eine gute Wirkung gegen Malaria besitzen.

Statt mit wässrigen Flüssigkeiten zu verdünnen, ist es auch möglich, die erfindungsgemäße Arzneimittelformulierung in anderer Form, z.B. als Pulver, Tabletten, Kapseln oder auch als Salbe herzustellen. In diesem Fall wird der Alkohol bevorzugt in kleinerer Menge zugegeben als bei der Herstellung der Formulierung zur Verwendung in flüssiger Form. Bevorzugt ist hier ein molares Mischungsverhältnis von Phospholipidverbindung : Alkohol von 1 : 5 bis 100. Gegebenenfalls kann der Alkohol aus dem Gemisch mindestens zum Teil wieder entfernt werden, um eine konzentrierte Formulierung zu erhalten. Dazu kann die Arzneimittelformulierung mit üblichen physiologisch verträglichen Füll-, Träger-, Verdünnungs- oder/und Hilfsstoffen vermischt werden und z.B. in Hohlzellen entsprechender Größe ausgegossen oder in Kapseln entsprechender Größe abgefüllt oder granuliert und dann gegebenenfalls unter Zusatz von weiteren üblichen Hilfsstoffen zu Tabletten verpresst werden. Die Formulierung kann beispielsweise mit einem oder mehreren der folgenden Hilfsstoffe gemischt werden: Stärke, Cellulose, Lactose, Formalin, Kasein, modifizierte Stärke, Magnesiumstearat, Calciumhydrogenphosphat, hochdisperse Kieselsäure, Talkum und Phenoxyethanol. Die erhaltene Mischung kann gegebenenfalls mit einer wässrigen Lösung aus beispielsweise Gelatine, Stärke, Polyvinylpyrrolidon, Vinylpyrrolidon-Vinylacetat-Copolymerisat oder/und Polyoxyethylensorbitatmonooleat granuliert und anschließend zu Tabletten verpresst oder in Kapseln abgefüllt werden.
Eine besonders nebenwirksame Ausführung dieser Wirkstoffe in Form von Tabletten, Kapseln usw. ist die Verwendung der Wirkstoffe in Kombination mit Cholesterin, ß-Sitosterin oder anderen Cholesterinanaloga in den für die Liposome beschriebenen molaren Verhältnissen.

Die erfindungsgemäßen Arzneimittelzubereitungen können insbesondere zur Behandlung oder/und Prophylaxe von Protozoenerkrankungen eingesetzt werden. Es hat sich gezeigt, dass die liposomalen Zubereitungen hervorragende Wirksamkeiten gegen Protozoen- und davon hervorgerufene Erkrankungen aufweisen und insbesondere geeignet sind gegen Plasmodien und damit für die Behandlung oder Prophylaxe von Malaria, gegen Trypanosomen und damit für die Behandlung oder Prophylaxe von Schlafkrankheit, gegen Amöben, z.B. Endamöben und Acanthamöben, für die Behandlung oder Prophylaxe von Amöbenruhr und Encephalitis und insbesondere gegen Leishmanien und damit für die Behandlung oder Prophylaxe von Leishmaniose. Neben der Behandlung von Menschen können die erfindungsgemäßen Arzneimittelzubereitungen vorteilhaft auch bei der Behandlung von Tieren und insbesondere bei Leishmaniose bei Hunden eingesetzt werden. Besonders bevorzugt werden die Arzneimittelformulierungen zur Behandlung von Leishmaniose oder/und zur Behandlung von durch Amöben hervorgerufene Erkrankungen eingesetzt. Bei der Behandlung von Leishmaniasis werden bevorzugt Formulierungen eingesetzt, welche als zusätzlichen Wirkstoff Amphotericin B enthalten.

Weiterhin hat sich gezeigt, dass die erfindungsgemäßen Arzneimittelformulierungen hervorragende Antitumormittel darstellen. Sie können somit zur Behandlung oder/und Prophylaxe von Krebs, insbesondere von Leukämie und soliden Tumoren eingesetzt werden. Auch bei der Behandlung von Krebs in Hunden haben sich die Substanzen durchaus bewährt. So konnten z.B. Morbus Cushing und Blasentumoren mit Erucylphospho-(N.N.N-trimethyl)-propylammonium mit gutem Erfolg behandelt werden.

Darüber hinaus können sie zur Stimulierung der Leukopoese sowie zur Behandlung von Erkrankungen, die durch Arthropoden verursacht werden und von Akarinosis eingesetzt werden.

Überraschenderweise hat sich herausgestellt, dass die erfindungsgemäße Arzneimittelformulierungen auch eine gute Wirksamkeit gegen Akarinosis, insbesondere Räude und gegen durch Arthropoden sowie durch Ascariden, wie etwa Milben oder Zecken verursachte Erkrankungen aufweist.

Zusätzliche Wirkstoffe können diese Indikationen gewünschtenfalls fördern, ergänzen oder erweitern. Insbesondere ein Zusatz von Amphotericin B zeigte eine synergistische Verstärkung der Wirksamkeit gegen Protozoenerkrankungen und eine Erweiterung auf systemische Pilzerkrankungen.

Weiterhin zeigen die erfindungsgemäßen Arzneimittelformulierungen auch eine hervorragende Wirksamkeit gegenüber bakteriellen Erkrankungen. Sie können deshalb auch zur Behandlung oder/und Prophylaxe von bakteriellen Erkrankungen, insbesondere zur Behandlung oder/und Prophylaxe von Ehrlichiose eingesetzt werden. Ehrlichiose ist eine bakterielle Erkrankung, die durch Zecken übertragen wird. Bei der Behandlung von Hunden mit erfindungsgemäßen Arzneimittelformulierungen wurde nicht nur eine deutliche Reduzierung des Ehrlichiosetiters festgestellt, sondern Heilung erreicht. Die Behandlung von Ehrlichiose kann in Kombination mit Tetracyclinen erfolgen.

Auch Augenerkrankungen, die mit zellulären Proliferationen einhergehen wie z.B. die proliferative Vitreoretinopathie oder Netzhautablösungen am Auge, die operativ nur schwer zu behandeln sind, und ebenfalls oftmals mit sehr stark proliferierenden Zellen einhergehen, können mit den erfindungsgemäßen Arzneimittelzubereitungen erfolgreich behandelt bzw. vermieden werden.

Die Behandlung der genannten Erkrankungen kann praktisch nebenwirkungsfrei durchgeführt werden. Überraschenderweise führt der Einsatz der erfindungsgemäßen Arzneimittelformulierungen nicht zu der bei Chemotherapien gefürchteten Immunsuppression, sondern sogar zu einer Stimulation der Leukopoese. Mit zunehmender Dauer der Therapie normalisiert sich auch das Blutbild. Mit anderen Worten, die hier beschriebenen Formulierungen mit Alkylphosphocholinen als Wirkstoff sind für Langzeittherapien sehr geeignet und ermöglichen bei therapeutisch effektiven Dosen eine nebenwirkungsfreie Behandlung. Die hierin besonders hervorgehobenen Alkylphosphocholine sind solche mit mindestens einer cis-Doppelbindung im Molekül wie z.B. Oleylphosphocholin, Erucylphosphocholin oder Erucylphospho-(N.N.N-trimethyl)-propylammonium. Alkylphosphocholine mit cis-Doppelbindungen zeichnen sich durch eine wesentlich größere therapeutische Breite aus, d.h., es können deutlich höhere Dosen appliziert werden als bei gesättigten Alkylphosphocholinen. Das ist dann besonders vorteilhaft, wenn die Erufosin-Therapie mit Strahlentherapie kombiniert wird. Mit Erufosin dotierte Tumorzellen sind gegenüber Strahlentherapie sensibilisiert und besonders anfällig.

Insbesondere in Kombination mit Cyclosporin A können die Zubereitungen auch zur immunsuppression eingesetzt werden.

Die erfindungsgemäßen Arzneimittelzubereitungen, insbesondere in Form von Liposomen werden bevorzugt zur Behandlung von Säugetieren und am meisten bevorzugt zur Behandlung von Hunden oder Menschen eingesetzt.

Es wurde überraschenderweise festgestellt, dass die erfindungsgemäßen Arzneimittelzubereitungen zur Behandlung oder/und Prophylaxe an Tieren, insbesondere bei Hunden hervorragend geeignet sind. Entsprechend werden die erfindungsgemäßen Arzneimittelzubereitungen bevorzugt in der Veterinärmedizin und dort insbesondere zur Behandlung von Tumor- und Protozoenerkrankungen eingesetzt. Insbesondere bei Hunden, bei denen bisherige, für den Menschen geeignete Mittel oftmals versagt haben, konnten mit den erfindungsgemäßen Zusammensetzungen hervorragende Ergebnisse erzielt werden. So können beispielsweise Leishmaniosen und Ehrlichiosen bei Tieren und insbesondere bei Hunden mit den erfindungsgemäßen Arzneimittelzubereitungen erfolgreich behandelt werden. Besonders bevorzugt wird hierbei Oleylphosphocholin als Wirkstoff und Ölsäure als negativer Ladungsträger eingesetzt.

Weiterhin wurde festgestellt, dass mit den erfindungsgemäßen Formulierungen auch Augenerkrankungen erfolgreich behandelt werden können, insbesondere Augenerkrankungen, die mit unkontrollierten zellulären Prozessen einhergehen.

Die vorliegende Erfindung betrifft neue Arzneimittelformulierungen, die als Wirkstoffe Alkylphosphocholine und Analoga, Alkyl-alkandiol-phoshocholine und Analoga sowie (Ether)- Lysolecithine und Analoga in verschiedenen Ausführungsformen enthalten. Die Wirkstoffe sind dabei integrale Bestandteile von Liposomen, die außerdem Cholesterin und Analoga sowie einen negativen Ladungsträger enthalten.

Die Arzneimittelformulierungen sind besonders geeignet zur Behandlung oder / und Prophylaxe von Krebs, von Protozoenerkrankungen wie Leishmaniosen und Amöbenerkrankungen, von Acariasis und von Erkrankungen, die durch Arthropoden verursacht werden sowie von bakteriellen Erkrankungen, wie z. B. Ehrlichiose, geeignet. Auch Augenerkrankungen, die mit unkontrollierten zellulären Prozessen einhergehen, können günstig beeinflusst werden.

Die folgenden Beispiele sollen die Erfindung veranschaulichen.

### Beispiele

### Beispielgruppe 1: Variation des Oleylphosphocholin/Cholesterin Verhältnisses

| | |
|---|---|
| Oleylphosphocholin | (MG 433,61) (Ol-PC) |
| Cholesterin | (MG 386,66) (Chol) |
| Ölsäure | (MG 282,47) |
| NaOH | (MG 40.00) |

### Beispiel 1 (a) Ol-PC, 40 mM; Chol, 35 mM

| | | |
|---|---|---|
| Ol-PC | 40,0 mM; 1,73 g | |
| Chol | 35,0 mM; 1,35 g | Einwaage: 100 g = 100 ml |
| Ölsäure | 10,0 mM; 0,283 g | |
| Na OH | 9,5 mM; 9,50 g 0,1 N NaOH | |

### Herstellung

Die Einwaage wird in 30 ml CH₂Cl₂ gelöst, CH₂Cl₂ abgezogen, getrocknet bis zur Gewichtskonstanz, mit 50 g 0.3 M Propandiol-1.2 und 9.5 g 0.1 N NaOH versetzt und dann so viel 0.3 M Propandiol-1.2 zugegeben, dass das Gesamtgewicht 100 g beträgt.
a) Tempern bei 55°C für 10 min
b) Ultraschall (100 %) bei 55° C für 20 min Noch warm wird durch 0,2 µ filtriert und bei +4 °C bis +8 °C gelagert.

### Bemerkungen - stabil, mindestens 1 Jahr bei +4 °C bis +8 °C.

### Beispiel 1 (b) Ol-PC, 40 mM: Chol. 40 mM

| | | |
|---|---|---|
| Ol-PC | 40,0 mM; 1,73 g | |
| Chol | 40,0 mM; 1,55 g | Einwaage: 100 g = 100 ml |
| Ölsäure | 10,0 mM; 0,283 g | |
| Na OH | 9,5 mM; 9,50 g 0,1 N Na OH | |

### Herstellung- wie bei Beispiel 1 (a)

### Bemerkungen- stabil, mindestens 1 Jahr bei +4 °C bis +8 °C.

### Beispiel 1 (c) Ol-PC, 40 mM; Chol. 45 mM

| | | |
|---|---|---|
| Ol-PC | 40,0 mM; 1,73 g | |
| Chol | 45,0 mM; 1,74 g | Einwaage: 100 g = 100 ml |
| Ölsäure | 10,0 mM; 0,283 g | |
| Na OH | 9,5 mM; 9,50 g 0,1 N NaOH | |

### Herstellung -wie Beispiel 1 (a)

### Bemerkungen - stabil, mindestens 1 Jahr bei + 4° C bis + 8° C.

Nach den Ergebnissen in den Beispielen 1 (a) bis 1 (c) kann ein Präparat zur subkutanen Behandlung von Hunden folgende Zusammensetzung besitzen:

| | | |
|---|---|---|
| | | mM |
| Ol-PC | (Wirkstoff) | 35 - 45 |
| Chol | (Hilfsstoff) | 35 - 45 |
| Ölsäure | (95 %) | 8 - 12 |

### Beispielgruppe 2 : Variation des Ölsäuregehaltes

Die Zusammensetzung wurde hinsichtlich des Ölsäuregehaltes variiert, allerdings stets mit einem Protonierungsgrad von 95 %. Diese Variationen wurden untersucht an

| | |
|---|---|
| Ol-PC | 40,0 mM |
| Chol | 40,0 mM |

Ölsäure als Natriumsalz wurde variiert zwischen 2.0 mM und 25 mM. Einige gut verwendbare und lagerfähige Formulierungen sind unter 2 (a) bis 2 (d) genau beschrieben.

| | |
|---|---|
| Oleylphosphocholin | (MG 433,61), |
| Cholesterin | (MG 386,66) |
| Ölsäure | (MG 282,47) |
| NaOH | (MG 40.00) |

### Beispiel 2 (a) Ol-PC, 40 mM: Chol. 40 mM; Ölsäure 16 mM

| | | |
|---|---|---|
| Ol-PC | 40,0 mM; 1,73 g | |
| Chol | 40,0 mM; 1,55 g | Einwaage: 100 g = 100 ml |
| Ölsäure | 16,0 mM; 0,452 g | |
| NaOH | 15,2 mM; 7,60 g 0,1 N NaOH | |

### Herstellung- wie bei Beispiel 1 (a)

### Bemerkungen- stabil, mindestens für 1 Jahr bei +4 °C bis +8 °C; nicht gewebereizend bei Hunden!

### Beispiel 2 (b) Ol-PC, 40 mM; Chol, 40 mM; Ölsäure 8 mM

| | | |
|---|---|---|
| Ol-PC | 40,0 mM; 1,73 g | |
| Chol | 40,0 mM; 1,55 g | Einwaage: 100 g = 100 ml |
| Ölsäure | 8,0 mM; 0,225 g | |
| NaOH | 7,6 mM; 7,60 g 0,1 N NaOH | |

### Herstellung- wie bei Beispiel 1 (a)

### Bemerkungen- stabil, für mindestens 1 Jahr bei +4 °C bis +8 °C; nicht gewebereizend bei Hunden!

### Beispiel 2 (c) Ol-PC, 40 mM; Chol, 40 mM; Ölsäure 6 mM

| | | |
|---|---|---|
| Ol-PC | 40,0 mM; 1,73 g | |
| Chol | 40.0 mM; 1,55 g | Einwaage: 100 g = 100 ml |
| Ölsäure | 6,0 mM; 0,170 g | |
| NaOH | 5,6 mM; 5,60 g 0,1 N NaOH | |

### Herstellung- wie bei Beispiel 1 (a)

### Bemerkungen- stabil, mindestens für 1 Jahr bei +4 °C bis +8 °C; nicht gewebereizend bei Hunden!

### Beispiel 2 (d) Ol-PC, 40 mM: Chol, 40 mM; Ölsäure 4 mM

| | | |
|---|---|---|
| Ol-PC | 40,0 mM; 1,73 g | |
| Chol | 40.0 mM; 1,55 g | Einwaage: 100 g = 100 ml |
| Ölsäure | 4,0 mM; 0,112 g | |
| NaOH | 3,8 mM; 3,80 g 0,1 N NaOH | |

### Herstellung- wie bei Beispiel 1 (a)

### Bemerkungen- stabil, mindestens für 1 Jahr bei +4 °C bis +8 °C; nicht gewebereizend bei Hunden!

### Beispielgruppe 3 : Herstellung von Ol-PC Dispersionen für die Anwendung in Tierversuchen

| | |
|---|---|
| Oleylphosphocholin (MG 433,61) | 39,20 mM |
| Cholesterin (MG 386,66) | 41,40 mM |
| Ölsäure (MG 282,47) | 5,66 mM |
| NaOH (MG 40,00) | 5,40 mM |

| | Einwaage | | |
|---|---|---|---|
| | pro 1kg | pro 0,5kg | pro 0,1kg |
| Ol-PC | 17,00 g | 8,5 g | 1,700 g |
| Chol | 16,00 g | 8,0 g | 1,600 g |
| Ölsäure | 1,60 g | 0,8 g | 0,160 g |
| NaOH, 0.1 N | 54,00 g | 27,0 g | 5,400 g |

### Beispiel 3 (a) Herstellung einer Probedispersion von 100 g Ol-PC

Die für 0,1 l = 100 ml notwendigen Substanzmengen werden in einen 500 ml Rundkolben eingewogen und in 40 ml CH₂Cl₂ vollständig gelöst. Die Lösung muss klar und partikelfrei sein. CH₂Cl₂ wird am Rotationsverdampfer unter leichtem Vakuum abgezogen und der Rückstand bis zur Gewichtskonstanz, am einfachsten über Nacht im Vakuum getrocknet: Ergebnis 3,44 bis 3,46 g, das entspricht > 99 % der Einwaage.

Man versetzt den Rückstand mit einer Lösung aus 90 ml 0,289 M Propandiol - 1,2 und 5,4 ml 0,1 M NaOH und bringt das Gesamtgewicht auf 100 g. Der dabei auftretende Volumenfehler mit 0,289 M Propandiol - 1,2 ist gering, denn die Dichte der Dispersion entspricht etwa 1. Man erwärmt unter Rotation auf 55 °C und hält das System bei dieser Temperatur:
a) Tempern bei 55° C für 10 min
b) Ultraschall (100%) bei 55° C für 20 min

Noch warm wird durch 0,2 sterilfiltriert und bei +4 °C bis +8 °C gelagert.

**Bemerkungen-** Die Dispersion ist stabil, Lagerung mindestens 1 Jahr. Sie erfüllt alle wichtigen Kriterien.
Lagerstabilität
Sterilfiltration möglich
Hitzesterilisation möglich
Synthese für Hilfsstoff entfällt
Hilfsstoff pharmazeutisch bekannt
Hilfsstoff hat außerdem Pufferkapazität bei pH 5,0.

### Beispiel 3 (b) Herstellung einer Dispersion von 500 g Ol - PC

Die für 0,5 l = 500 ml notwendigen Substanzmengen werden in einen 1 l Rundkolben eingewogen und in 100 ml CH₂Cl₂ vollständig gelöst. Die Lösung muss klar und partikelfrei sein. CH₂Cl₂ wird am Rotationsverdampfer unter leichtem Vakuum abgezogen und der Rückstand bis zur Gewichtskonstanz, am einfachsten über Nacht im Vakuum getrocknet: Ergebnis: 17,2 bis 17,3 g, das entspricht > 99 % der Einwaage.

Man versetzt den Rückstand mit einer Lösung aus 450 ml 0,289 M Propandiol - 1,2 und 27 ml 0,1 N NaOH und bringt das Gesamtgewicht auf 500 g. Das spez. Gewicht der Dispersion ist fast 1, sodass der Volumenfehler gering ist. Man erwärmt unter Rotation auf 55 °C und hält das System mit einem Wasserbad bei dieser Temperatur:
a) Tempern bei 55° C für 10 min
b) Ultraschall (100%) bei 55° C für 20 min

Noch warm wird durch 0,2 µ sterilfiltriert und bei +4 °C bis +8 °C gelagert.

**Bemerkungen-** Die Dispersion ist stabil, Lagerung mindestens 1 Jahr. Sie erfüllt alle wichtigen Kriterien.
Lagerstabilität
Sterilfiltration möglich
Hitzesterilisation möglich
Synthese für Hilfsstoff entfällt
Hilfsstoff pharmazeutisch bekannt
Hilfsstoff hat außerdem Pufferkapazität bei pH 5,0.

### Vergleichs-Beispielgruppe 4: Fettsäureamide von Aminosäuren als negative Ladungsträger

| | |
|---|---|
| Oleylphosphocholin | (MG 433.61) |
| Cholesterin | (MG 386.66) |
| N-Oleoyl-alanin, Na⁽⁺⁾-Salz | (MG 375.53) |

### Beispiel 4 (a) Ol-PC, 40 mM: Chol, 40 mM: N-Oleovl-alanin. 5 mM

| | | |
|---|---|---|
| Ol-PC | 40,0 mM; 1,73 g | |
| Chol | 40,0 mM; 1,55 g | Einwaage: 100 g = 100 ml |
| N-Oleoyl-Al | 5,0 mM; 0,19 g | |

### Herstellung

Die Einwaage wird in 30 ml CH₂ Cl₂ gelöst, das Lösungsmittel abgezogen und der Rückstand bis zur Gewichtskonstanz getrocknet. Man bringt den Rückstand mit 0.3 M Propandiol-1.2 auf insgesamt 100 g und erwärmt auf 55° C:
a) Tempern bei 55° C für 10 min
b) Ultraschall (100 %) bei 55° C für 20 min.

Noch warm wird durch 0.2µ steril filtriert und bei + 4° C bis +8° C gelagert.

### Beispiel 4 (b) Ol-PC, 40 mM: Chol, 40 mM; N-Oleoyl-alanin, 10 mM

| | | |
|---|---|---|
| Ol-PC | 40,0 mM; 1,73 g | |
| Chol | 40,0 mM; 1,55 g | Einwaage: 100 g = 100 ml |
| N-Oleoyl-Al | 10,0 mM; 0,38 g | |

### Herstellung

Die Einwaage wird in 30 ml CH₂ Cl₂ gelöst, das Lösungsmittel abgezogen und der Rückstand bis zur Gewichtskonstanz getrocknet. Man bringt den Rückstand mit 0.3 M Propandiol-1.2 auf insgesamt 100 g und erwärmt auf 55° C:
a) Tempern bei 55° C für 10 min
b) Ultraschall (100 %) bei 55° C für 20 min.

Noch warm wird durch 0.2µ steril filtriert und bei + 4° C bis +8° C gelagert.

### Vergleichs-Beispielgruppe 5: Fettsäureamide von Glycero-phospho-ethanolamin als negative Ladungsträger

| | |
|---|---|
| Oleylphosphocholin | (MG 433.61) |
| Cholesterin | (MG 386.66) |
| N-Oleoyl-glycero-phospho-ethanolamid, Na⁽⁺⁾-Salz | (MG 501.57) |

### Beispiel 5 (a) Ol-PC, 40 mM: Chol: 40 mM: N-Oleovl-GPE. 5 mM

| | | |
|---|---|---|
| Ol-PC | 40,0 mM; 1,73 g | |
| Chol | 40,0 mM; 1,55 g | Einwaage: 100 g = 100 ml |
| N-Oleoyl-GPE | 5,0 mM; 0,25 g | |

### Herstellung

Die Einwaage wird in 30 ml CH₂Cl₂ gelöst, das Lösungsmittel abgezogen und der Rückstand bis zur Gewichtskonstanz getrocknet. Man bringt den Rückstand mit 0.3 M Propandiol-1.2 auf insgesamt 100 g und erwärmt auf 55° C:
a) Tempern bei 55° C für 10 min
b) Ultraschall (100 %) bei 55° C für 20 min.

Noch warm wird durch 0.2µ steril filtriert und bei + 4° C bis +8° C gelagert.

### Beispiel 5 (b) Ol-PC, 40 mM: Chol, 40 mM: N-Oleoyl-GPE, 10 mM

| | | |
|---|---|---|
| Ol-PC | 40,0 mM; 1,73 g | |
| Chol | 40,0 mM; 1,55 g | Einwaage: 100 g = 100 ml |
| N-Oleoyl-GPE | 10,0 mM; 0,50 g | |

### Herstellung

Die Einwaage wird in 30 ml CH₂Cl₂ gelöst, das Lösungsmittel abgezogen und der Rückstand bis zur Gewichtskonstanz getrocknet. Man bringt den Rückstand mit 0.3 M Propandiol-1.2 auf insgesamt 100 g und erwärmt auf 55° C:
a) Tempern bei 55° C für 10 min
b)Ultraschall(100 %) bei 55° C für 20 min.

Noch warm wird durch 0.2µ steril filtriert und bei + 4° C bis +8° C gelagert.

## Patentansprüche

1. Arzneimittelzubereitung, enthaltend als Wirkstoff a) eine Phospholipidverbindung der allgemeinen Formel I: 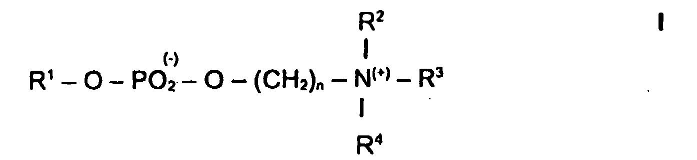 worin R¹ ein gesättigter oder ungesättigter Kohlenwasserstoffrest mit 15 bis 26 C-Atomen ist
worin R^{2,} R³ und R⁴ bei jedem Auftreten jeweils unabhängig H, eine C, bis C₆-Alkylgruppe, eine C₃ bis C₆-Cycloalkylgruppe oder eine C₂ bis C₆-Hydroxyalkylgruppe sind, wobei zwei aus R², R³ und R⁴ miteinander eine C₂ bis C₅-Alkylengruppe bilden können,
worin n eine ganze Zahl von 2 bis 6 ist,
oder eine Phospholipidverbindung der allgemeinen Formel II (Alkylphosphocholine mit Ring-Stickstoff): 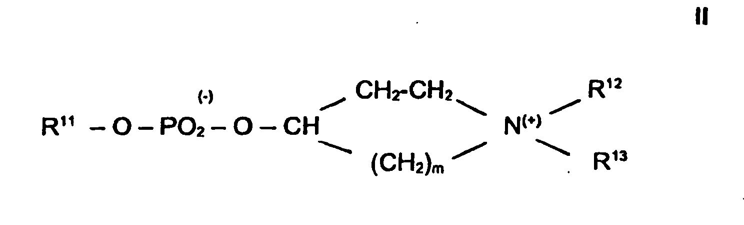 worin R¹¹ ein gesättigter, ungesättigter oder auch mehrfach ungesättigter Kohlenwasserstoffrest mit 15 bis 26 C-Atomen ist,
worin R¹² und R¹³ bei jedem Auftreten jeweils unabhängig H, eine C₁ bis C₆-Alkylgruppe oder eine C₂ bis C₆-Hydroxyalkylgruppe sind,
worin m eine ganze Zahl von 1 oder 2 ist,
oder eine Phospholipidverbindung der allgemeinen Formel III (Alkyl-ethylenglycol-phosphocholine) 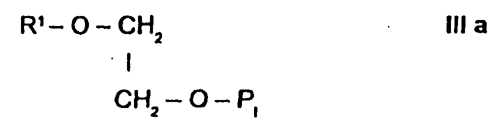 oder 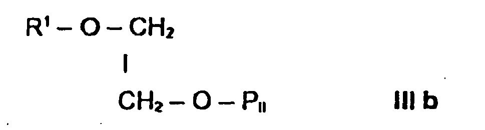 worin P_{I} 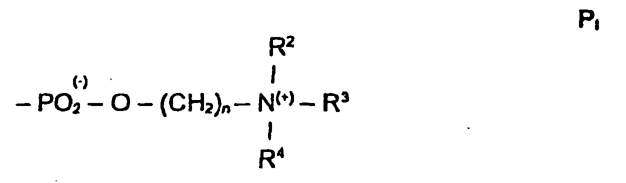 darstellt und P_{II} 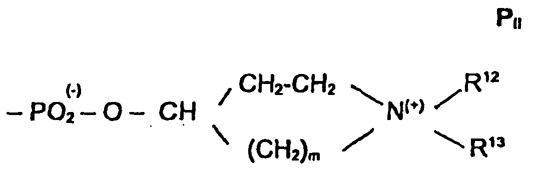 darstellt
oder eine Phospholipidverbindung der allgemeinen Formel IV (Alkyl-alkandiol-phosphocholine) 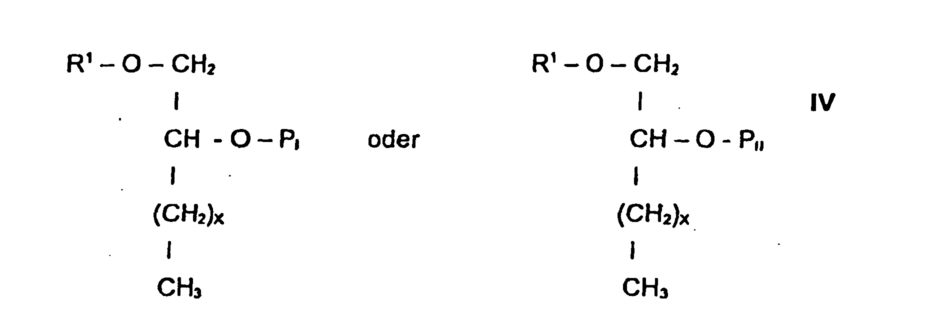 worin die Reste R¹ und P_{I} oder P_{II} auch gegeneinander ausgetauscht werden können,
worin R¹, P_{I} und P_{II} den obigen Bedeutungen entsprechen,
worin x eine ganze Zahl von 0 bis 4 ist,
oder eine Phospholipidverbindung der allgemeinen Formel V (Ether-lysolecithine) 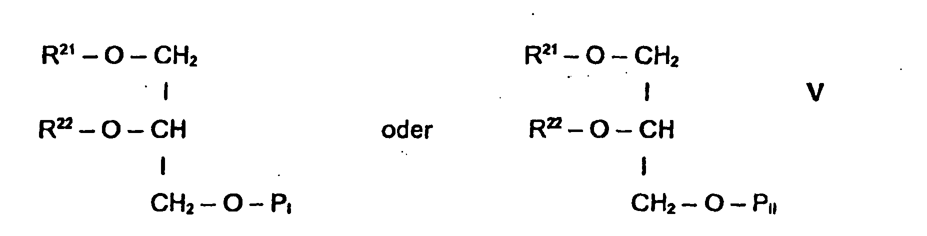 worin R²¹ ein gesättigter oder ein einfach oder mehrfach ungesättigter Kohlenwasserstoffrest mit 15 bis 26 C-Atomen ist,
worin R²² eine C₁ bis C₆-Alkylgruppe bedeutet,
worin P_{I} und P_{II} den obigen Bedeutungen entsprechen, wobei die Reste R²¹, R²² und P_{I} bzw. P_{II} beliebig über die Positionen im Glycerinmolekül verteilt werden können
oder eine Phospholipidverbindung der allgemeinen Formel VI (Alkyl-substituierte Alkandiol-phosphocholine): 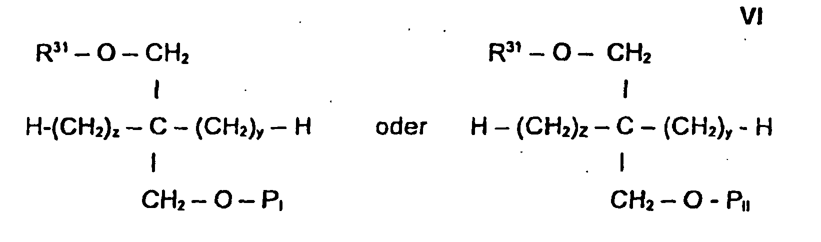 worin R³¹ ein gesättigter oder einfach oder mehrfach ungesättigter Kohlenwasserstoffrest mit 15 bis 26 C-Atomen ist,
worin y und z unabhängig voneinander eine ganze Zahl von 0 bis 3 sein können, aber y und z nicht beide gleichzeitig 0 sein können,
worin P_{I} und P_{II} den obigen Bedeutungen entsprechen,
oder eine Phospholipidverbindung der allgemeinen Formel VII (Alkyl-2-Methylpropandiol-(1,3)-phosphocholin) 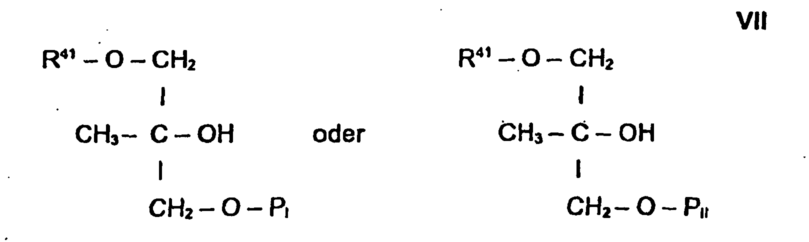 worin R⁴¹ ein gesättigter oder einfach oder mehrfach ungesättigter Kohlenwasserstoffrest mit 15 bis 26 C-Atomen ist,
worin P_{I} und P_{II} die oben angegebenen Bedeutungen haben
**dadurch gekennzeichnet,**
**dass** die Zusammensetzung weiterhin den Bestandteil
b) umfassend Cholesterin, 7 ß-Hydroxycholesterin oder/und ein ß-Sitosterin sowie als Bestandteil
c) einen negativen Ladungsträger, ausgewählt aus
einer Carbonsäure mit 16 bis 36 C-Atomen, die bevorzugt eine gesättigte, einfach ungesättigte und mehrfach ungesättigte Fettsäure ist und besonders bevorzugt cis-Doppelbindungen enthält.

2. Arzneimittel nach Anspruch 1,
**dadurch gekennzeichnet,**
**dass** das Mittel in Form von Liposomen vorliegt.

3. Arzneimittelzubereitung nach Anspruch 1 oder 2,
**dadurch gekennzeichnet,**
**dass** sie 10 bis 60 Mol-% des Wirkstoffs a)
10 bis 65 Mol-% der Verbindung b) und
3 bis 30 Mol-% des negativen Ladungsträgers c) enthält.

4. Arzneimittelzubereitung nach einem der vorhergehenden Ansprüche,
**dadurch gekennzeichnet,**
**dass** R¹, R¹¹, R²¹, R³¹ oder/und R⁴¹ ein C₁₅-C₂₄-Alkylrest, ein C₁₅-C₂₄-Alkenylrest, ein C₁₅-C₂₄-Alkadienylrest oder ein C₁₅-C₂₄-Alkatrienylrest ist.

5. Arzneimittelzubereitung nach einem der vorhergehenden Ansprüche,
**dadurch gekennzeichnet,**
**dass** n = 2 ist.

6. Arzneimittelformulierung nach einem der vorhergehenden Ansprüche,
**dadurch gekennzeichnet,**
**dass** R², R³ und R⁴ bei jedem Auftreten ein Methylrest ist.

7. Arzneimittelzubereitung nach einem der vorhergehenden Ansprüche,
**dadurch gekennzeichnet,**
**dass** sie als Wirkstoff 1-O-Octadecyl-2-O-methyl-glycero-3-phosphocholin (ET18OCH3) enthält.

8. Arzneimittelzubereitung nach einem der vorhergehenden Ansprüche,
**dadurch gekennzeichnet,**
**dass** R^{1,} R¹¹, R²¹, R³¹ bzw. R⁴¹ Oleyl darstellt.

9. Arzneimittelzubereitung nach einem der vorhergehenden Ansprüche,
**dadurch gekennzeichnet,**
**dass** der negative Ladungsträger ausgewählt ist aus Ölsäure, Linolsäure, Laurinsäure oder/und Palmitinsäure.

10. Arzneimittelformulierung nach einem der vorhergehenden Ansprüche,
**dadurch gekennzeichnet,**
**dass** sie weiterhin ein pharmakologisch geeignetes Träger- oder/und Verdünnungsmittel enthält.

11. Arzneimittelzubereitung nach einem der vorhergehenden Ansprüche,
**dadurch gekennzeichnet,**
**dass** die Komponenten a), b) und c) zusammen 100 Mol-% ergeben.

12. Arzneimittelzubereitung nach einem der vorhergehenden Ansprüche,
**dadurch gekennzeichnet,**
**dass** sie die Verbindung der Formel I in einer Menge von 0,1 bis 200 µmol/g enthält.

13. Arzneimittelzubereitung nach einem der vorhergehenden Ansprüche,
**dadurch gekennzeichnet,**
**dass** sie in einer zur intravenösen, oralen oder subkutanen Verabreichung geeigneten Form vorliegt und für den Fall einer oralen Verabreichung als Tablette oder Kapsel formuliert ist.

14. Verfahren zur Herstellung einer Arzneimittelzubereitung nach einem der vorhergehenden Ansprüche,
**dadurch gekennzeichnet,**
**dass** man
a) eine Verbindung a)
als Wirkstoff mit einer Verbindung b) und einer Verbindung c) mischt.

15. Verwendung einer Zusammensetzung gemäß einem der Ansprüche 1 bis 13 zur Herstellung eines Arzneimittels zur Stimulierung der Leukopoese oder/und zur Behandlung oder/und Prophylaxe von Acarinoxis und von durch Atropoden verursachten Erkrankungen oder zur Behandlung oder/und Prophylaxe von Tumorerkrankungen oder zur Behandlung oder/und Prophylaxe von Protozoenerkrankungen insbesondere von Leishmaniosen oder/und Amöbenerkrankungen, oder zur Behandlung oder/und Prophylaxe von durch Aschariden, insbesondere Milben oder Zecken verursachten Erkrankungen, insbesondere von Räude oder zur Behandlung oder/und Prophylaxe von bakteriellen Erkrankungen, insbesondere von Erlichiose oder zur Behandlung oder/und Prophylaxe von Augenerkrankungen, die mit zellulären Proliferationen einhergehen, insbesondere von Netzhautablösungen.

16. Verwendung einer Zusammensetzung gemäß einem der Ansprüche 1 bis 13 zur Herstellung einer Arzneimittelzubereitung für die Veterinärmedizin.

17. Verwendung nach Anspruch 16 zur Behandlung oder/und Prophylaxe von Tumor- oder/und Protozoenerkrankungen.

18. Arzneimittelzubereitung nach einem der Ansprüche 1 bis 13, insbesondere für den Einsatz in der Veterinärmedizin,
**dadurch gekennzeichnet,**
**dass** sie als Wirkstoff a) Oleylphosphocholin, als Bestandteil b) Cholesterin, 7ß-Hydroxycholesterin oder/und ein ß-Sitostein und als negativen Ladungsträger c) Ölsäure enthält.

19. Arzneimittelzubereitung nach einem der Ansprüche 1 bis 13,
**dadurch gekennzeichnet,**
**dass** sie liposomale Alkylphosphocholine enthält und zur oralen Verabreichung vorgesehen ist.

20. Arzneimittelzubereitung nach einem der Ansprüche 1-13,
**dadurch gekennzeichnet,**
**dass** sie einen weiteren Wirkstoff umfasst, ausgewählt aus Oxytetracyclin, Doxycyclin, Minocyclin, Amphotericin B, Griseofulvin, Cyclosporin oder Artemether.

21. Arzneimittelzubereitung nach einem der Ansprüche 1-13,
**dadurch gekennzeichnet,**
**dass** sie als weiteren Wirkstoff Amphotericin B umfasst.

## Claims

1. A medicinal preparation containing as active ingredient a) a phospholipid compound of the general formula I: 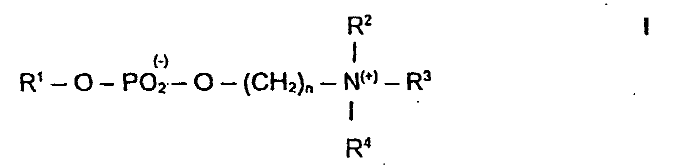 in which R¹ is a saturated or unsaturated hydrocarbon residue with 15 to 26 C atoms
in which, on each occurrence, R², R³ and R⁴ are in each case independently H, a C₁ to C₆ alkyl group, a C₃ to C₆ cycloalkyl group or a C₂ to C₆ hydroxyalkyl group, two of R², R³ and R⁴ being capable of forming a C₂ to C₅ alkylene group with one another,
in which n is an integer from 2 to 6,
or a phospholipid compound of the general formula II (alkylphosphocholines with ring nitrogen) : 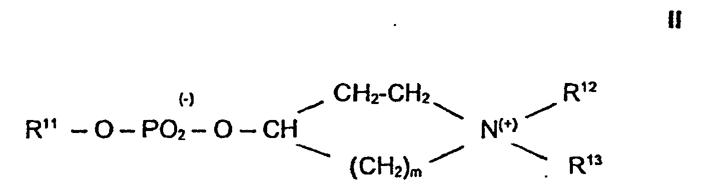 in which R¹¹ is a saturated, unsaturated or also polyunsaturated hydrocarbon residue with 15 to 26 C atoms,
in which, on each occurrence, R¹² and R¹³ are in each case independently H, a C₁ to C₆ alkyl group or a C₂ to C₆ hydroxyalkyl group,
in which m is an integer of 1 or 2,
or a phospholipid compound of the general formula III (alkyl ethylene glycol phosphocholines) 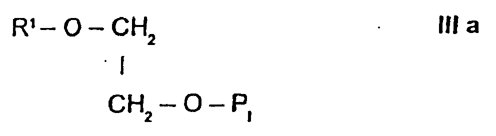 or 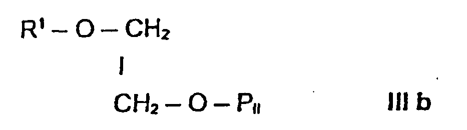 in which P_{I} represents 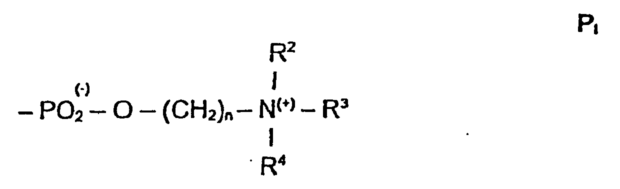 and P_{II} represents 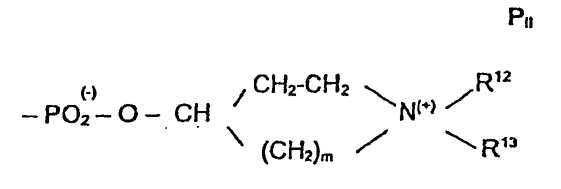 or a phospholipid compound of the general formula IV (alkyl alkanediol phosphocholines) 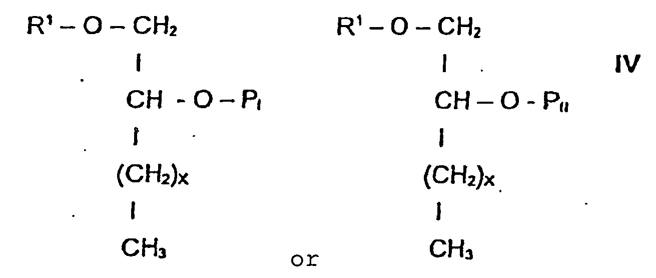 in which the residues R¹ and P_{I} or P_{II} may also be interchanged,
in which R¹, P_{I} and P_{II} correspond to the above meanings,
in which x is an integer from 0 to 4,
or a phospholipid compound of the general formula V (ether lysolecithins) 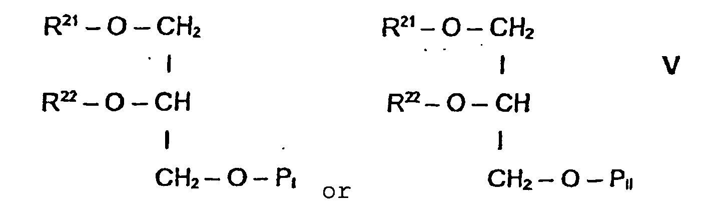 in which R²¹ is a saturated or a mono- or polyunsaturated hydrocarbon residue with 15 to 26 C atoms,
in which R²² means a C₁ to C₆ alkyl group,
in which P_{I} and P_{II} correspond to the above meanings, it being possible for the residues R²¹, R²² and P_{I} or P_{II} to be distributed at will among the positions in the glycerol molecule
or a medicinal preparation containing as active ingredient a phospholipid compound of the general formula VI (alkyl-substituted alkanediol phosphocholines): 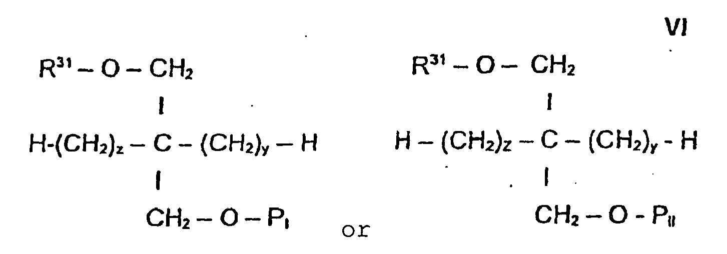 in which R³¹ is a saturated or mono- or polyunsaturated hydrocarbon residue with 15 to 26 C atoms,
in which y and z may mutually independently be an integer from 0 to 3, but y and z cannot both simultaneously be 0,
in which P_{I} and P_{II} correspond to the above meanings,
or a phospholipid compound of the general formula VII (alkyl 2-methyl 1,3-propanediol phosphocholine) 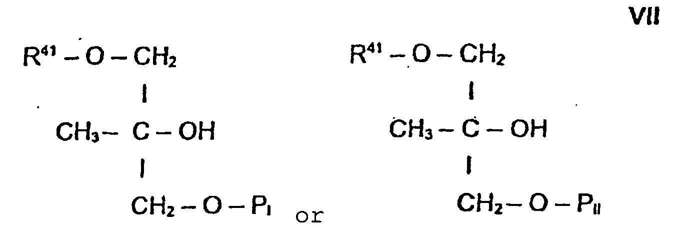 in which R⁴¹ is a saturated or mono- or polyunsaturated hydrocarbon residue with 15 to 26 C atoms.
in which P_{I} and P_{II} have the above-stated meanings, **characterised in that**
the composition furthermore contains the constituent
b) comprising cholesterol, 7β-hydroxycholesterol and/or a β-sitosterol and as constituent
c) a negative charge carrier selected from a carboxylic acid with 16 to 36 C atoms, which is preferably a saturated, monounsaturated and polyunsaturated fatty acid and particularly preferably contains cis double bonds.

2. A medicament according to claim 1, **characterised in that** the agent assumes the form of liposomes.

3. A medicinal preparation according to claim 1 or claim 2, **characterised in that** it contains
10 to 60 mol% of active ingredient a)
10 to 65 mol% of compound b) and
3 to 30 mol% of the negative charge carrier c).

4. A medicinal preparation according to any one of the preceding claims, **characterised in that** R¹, R¹¹, R²¹, R³¹ and/or R⁴¹ is/are a C₁₅-C₂₄ alkyl residue, a C₁₅-C₂₄ alkenyl residue, a C₁₅-C₂₄ alkadienyl residue or a C₁₅-C₂₄ alkatrienyl residue.

5. A medicinal preparation according to any one of the preceding claims, **characterised in that** n = 2.

6. A medicinal formulation according to any one of the preceding claims, **characterised in that**, on each occurrence, R², R³ and R⁴ is a methyl residue.

7. A medicinal preparation according to any one of the preceding claims, **characterised in that** it contains 1-O-octadecyl-2-O-methyl-glycerol-3-phosphocholine (ET180CH3) as active ingredient.

8. A medicinal preparation according to any one of the preceding claims, **characterised in that** R¹, R¹¹, R²¹, R³¹ or R⁴¹ represent oleyl.

9. A medicinal preparation according to any one of the preceding claims, **characterised in that** the negative charge carrier is selected from oleic acid, linoleic acid, lauric acid and/or palmitic acid.

10. A medicinal formulation according to any one of the preceding claims, **characterised in that** it furthermore contains a pharmacologically suitable carrier substance and/or diluent.

11. A medicinal preparation according to any one of the preceding claims, **characterised in that** components a), b) and c) together add up to 100 mol%.

12. A medicinal preparation according to any one of the preceding claims, **characterised in that** it contains the compound of the formula I in a quantity of 0.1 to 200 µol/g.

13. A medicinal preparation according to any one of the preceding claims, **characterised in that** it assumes a form suitable for intravenous, oral or subcutaneous administration and, in the case of oral administration, is formulated as a tablet or capsule.

14. A method for producing a medicinal preparation according to any one of the preceding claims, **characterised in that**
a) a compound a) as active ingredient is mixed with a compound b) and a compound c).

15. Use of a composition according to any one of claims 1 to 13 for producing a medicament for the stimulation of leukopoiesis and/or for the treatment and/or prevention of acariasis and of diseases caused by arthropods or for the treatment and/or prevention of tumour diseases or for the treatment and/or prevention of protozoan diseases, in particular of leishmaniases and/or amoebic diseases, or for the treatment and/or prevention of diseases caused by ascarides, in particular mites or ticks, in particular of mange or for the treatment and/or prevention of bacterial diseases, in particular of ehrlichiosis or for the treatment and/or prevention of eye diseases associated with cellular proliferation, in particular of retinal detachment.

16. Use of a composition according to any one of claims 1 to 13 for producing a medicinal preparation for veterinary medicine.

17. Use according to claim 16 for the treatment and/or prevention of tumour diseases and/or protozoan diseases.

18. A medicinal preparation according to any one of claims 1 to 13, in particular for use in veterinary medicine, **characterised in that** it contains oleylphosphocholine as active ingredient a), cholesterol, 7β-hydroxycholesterol and/or a β-sitosterol as constituent b) and oleic acid as negative charge carrier c).

19. A medicinal preparation according to any one of claims 1 to 13, **characterised in that** it contains liposomal alkylphosphocholines and is intended for oral administration.

20. A medicinal preparation according to any one of claims 1-13, **characterised in that** it comprises a further active ingredient selected from oxytetracycline, doxycycline, minocycline, amphotericin B, griseofulvin, cyclosporin or artemether.

21. A medicinal preparation according to any one of claims 1-13, **characterised in that** it comprises amphotericin B as a further active ingredient.

## Revendications

1. Composition pharmaceutique, contenant comme agent actif a), un composé phospholipide de la formule générale I : 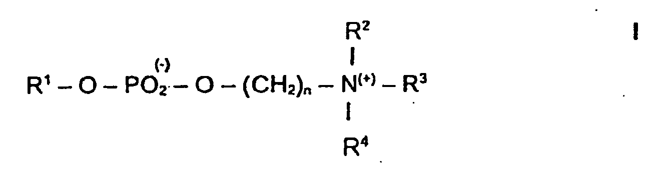 où R¹ est un reste hydrocarbure saturé ou insaturé, ayant 15 à 26 atomes C,
où R², R³ et R⁴ sont à chaque occurrence, chaque fois indépendamment, H, un radical alkyle en C₁ à C₆, un radical cycloalkyle en C₃ à C₈ ou un radical hydroxyalkyle en C₂ à C₆, où deux parmi R², R³ et R⁴ peuvent former l'un avec l'autre, un radical alkylène en C₂ à C₅,
où n est un nombre entier allant de 2 à 6,
ou un composé phospholipide de la formule générale II (alkylphosphocholine avec anneau azoté) : 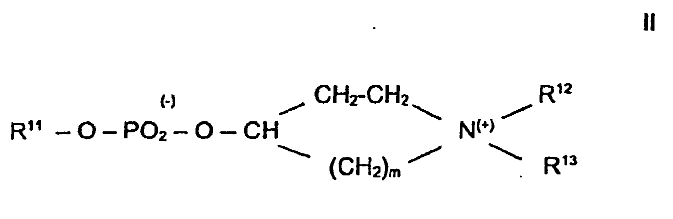 où R¹¹ est un reste hydrocarbure saturé, insaturé ou plusieurs fois insaturé, ayant 15 à 26 atomes C,
où R¹² et R¹³ sont à chaque occurrence, chaque fois indépendamment, H, un radical alkyle en C₁ à C₆ ou un radical hydroxyalkyle en C₂ à C₆,
où m est le nombre entier 1 ou 2,
ou un composé phospholipide de la formule générale III (alkyléthylèneglycolphosphocholine) : 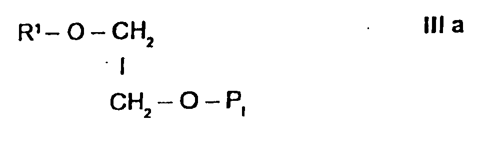 ou 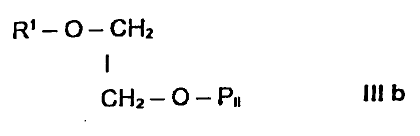 où P_{I} représente : 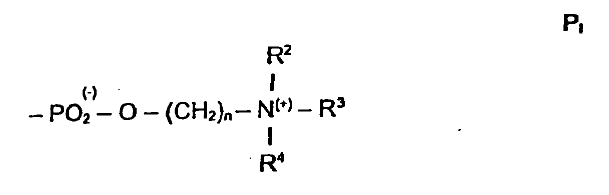 et P_{II} représente : 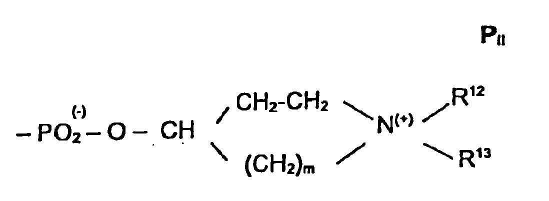 ou un composé phospholipide de la formule générale IV (alkylalcanediolphosphocholine) : 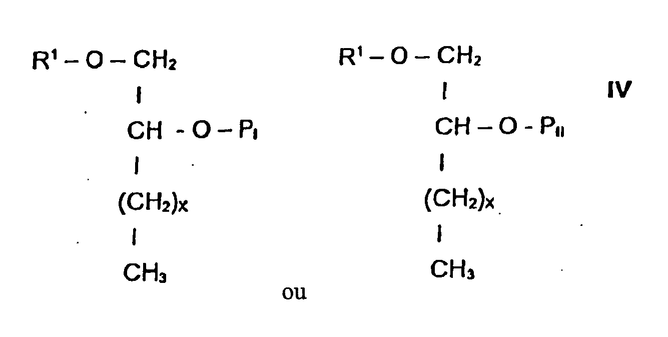 où les restes R¹ et P_{I} ou P_{II} peuvent être échangés l'un avec l'autre,
où R¹, P_{I} et P_{II} ont les significations ci-dessus,
où x est un nombre entier allant de 0 à 4,
ou un composé phospholipide de la formule générale V (éther-lysolécithine) : 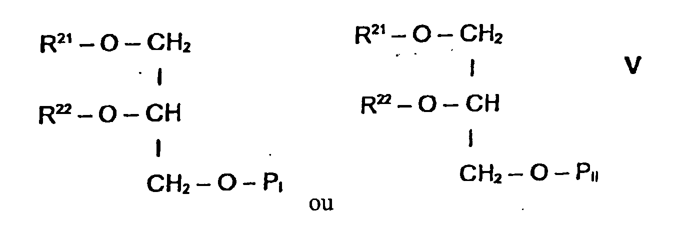 où R²¹ est un reste hydrocarbure saturé ou une ou plusieurs fois insaturé, ayant 15 à 26 atomes C,
où R²² est un radical alkyle en C₁ à C₆,
où P_{I} et P_{II} ont les significations ci-dessus, où les restes R²¹, R²² et P_{I} et respectivement, P_{II} peuvent être distribués de manière quelconque sur les positions de la molécule de glycérine,
ou un composé phospholipide de la formule générale VI (alcanediolphosphocholine substitué par alkyle) : 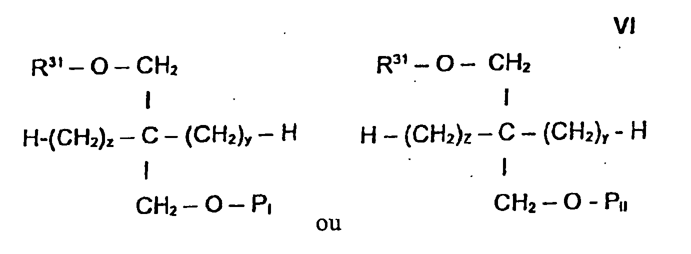 où R³¹ est un reste hydrocarbure saturé ou une ou plusieurs fois insaturé, ayant 15 à 26 atomes C,
où y et z peuvent être indépendamment l'un de l'autre, un nombre entier allant de 0 à 3, mais y et z ne peuvent pas être simultanément 0,
où P_{I} et P_{II} ont les significations ci-dessus,
ou un composé phospholipide de la formule générale VII (alkyl-2-méthylpropanediol-(1,3)-phosphocholine) : 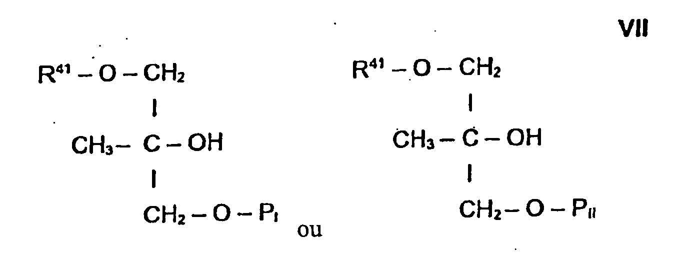 où R⁴¹ est un reste hydrocarbure saturé ou une ou plusieurs fois insaturé, ayant 15 à 26 atomes C,
où P_{I} et P_{II} ont les significations ci-dessus,
**caractérisée en ce que** la composition contient en outre, le constituant b) comprenant le cholestérol, le 7β-hydroxycholestérol et/ou un β-sitostérol, ainsi que comme constituant c), un porteur de charge négative, choisi parmi un acide carboxylique ayant 16 à 36 atomes C, qui est de préférence, un acide gras saturé, une fois insaturé ou plusieurs fois insaturé et qui contient de préférence, des doubles liaisons cis.

2. Agent pharmaceutique selon la revendication 1, **caractérisé en ce que** l'agent se présente sous forme de liposomes.

3. Composition pharmaceutique selon la revendication 1 ou 2, **caractérisée en ce qu'**elle contient 10 à 60 % en moles de l'agent actif a), 10 à 65 % en moles du composé b) et 3 à 30 % en moles du porteur de charge négative c).

4. Composition pharmaceutique selon l'une des revendications précédentes, **caractérisée en ce que** R¹, R¹¹, R²¹, R³¹ et/ou R⁴¹ est un reste alkyle en C₁₅-C₂₄, un reste alcényle en C₁₅-C₂₄, un reste alcadiényle en C₁₅-C₂₄ ou un reste alcatriényle en C₁₅-C₂₄.

5. Composition pharmaceutique selon l'une des revendications précédentes, **caractérisée en ce que** n = 2.

6. Composition pharmaceutique selon l'une des revendications précédentes, **caractérisée en ce que** R², R³ et R⁴ sont chaque fois, un reste méthyle.

7. Composition pharmaceutique selon l'une des revendications précédentes, **caractérisée en ce qu'**elle contient comme agent actif, la 1-O-octadécyl-2-O-méthylglycéro-3-phosphocholine (ET18OCH3).

8. Composition pharmaceutique selon l'une des revendications précédentes, **caractérisée en ce que** R¹, R¹¹, R²¹, R³¹ ou R⁴¹ représente oléyle.

9. Composition pharmaceutique selon l'une des revendications précédentes, **caractérisée en ce que** le porteur de charge négative est choisi parmi l'acide oléique, l'acide linoléique, l'acide laurique et/ou l'acide palmitique.

10. Formulation pharmaceutique selon l'une des revendications précédentes, **caractérisée en ce qu'**elle contient en outre un support et/ou diluant pharmacologiquement approprié.

11. Composition pharmaceutique selon l'une des revendications précédentes, **caractérisée en ce que** les composants a), b) et c) donnent ensemble, 100 % en moles.

12. Composition pharmaceutique selon l'une des revendications précédentes, **caractérisée en ce qu'**elle contient le composé de formule II en une quantité allant de 0,1 à 200 µmole/g.

13. Composition pharmaceutique selon l'une des revendications précédentes, **caractérisée en ce qu'**elle se présente sous une forme appropriée pour une administration intraveineuse, orale ou sous-cutanée et dans le cas d'une formulation orale, elle est formulée sous forme d'un comprimé ou d'une capsule.

14. Procédé de préparation d'une composition pharmaceutique selon l'une des revendications précédentes, **caractérisé en ce que** l'on mélange un composé a) comme agent actif avec un composé b) et un composé c).

15. Utilisation d'une composition selon l'une des revendications 1 à 13, pour la préparation d'un médicament pour stimuler la leucopoïèse et/ou pour le traitement et/ou la prophylaxie de l'acarinose et de maladies engendrées par des atropodes ou pour le traitement et/ou la prophylaxie de maladies tumorales ou pour le traitement et/ou la prophylaxie de maladies dues à des protozoaires, en particulier la leishmaniose et/ou les maladies dues aux amibes, ou pour le traitement et/ou la prophylaxie de maladies engendrées par des acaris, en particulier acariens et tiques, en particulier la gale, ou pour le traitement et/ou la prophylaxie de maladies bactériennes, en particulier l'erlichiose, ou pour le traitement et/ou la prophylaxie de maladies des yeux, qui s'accompagnent de proliférations cellulaires, en particulier des décollements de rétine.

16. Utilisation d'une composition selon l'une des revendications 1 à 13, pour la préparation d'un médicament pour la médecine vétérinaire.

17. Utilisation selon la revendication 16, pour le traitement et/ou la prophylaxie de maladies tumorales et/ou dues à des protozoaires.

18. Composition pharmaceutique selon l'une des revendications 1 à 13, en particulier pour une mise en oeuvre en médecine vétérinaire, **caractérisée en ce qu'**elle contient comme agent actif a), l'oléylphosphocholine, comme constituant b), le cholestérol, le 7β-hydroxycholestérol et/ou un β-sitostérol, et comme porteur de charge négative c), l'acide oléique.

19. Composition pharmaceutique selon l'une des revendications 1 à 13, **caractérisée en ce que**lle contient une alkylphosphocholine liposomiale et qu'elle est prévue pour une administration orale.

20. Composition pharmaceutique selon l'une des revendications 1 à 13, **caractérisée en ce que**lle contient un autre agent actif, choisi parmi l'oxytétracycline, la doxycycline, la minocycline, l'amphotéricine B, la griséofulvine, la cyclosporine ou l' artéméther.

21. Composition pharmaceutique selon l'une des revendications 1 à 13, **caractérisée en ce que**lle contient comme autre agent actif, l'amphotéricine B.
